# FASCICULE DE BREVET EUROPEEN

(11) **EP 2 027 169 B1**
(45) Date de publication et mention de la délivrance du brevet: **14.08.2013**
(21) Numéro de dépôt: 07734792.0
(22) Date de dépôt: 01.06.2007
(51) Int. Cl.: C08F 230/02

(54) **PROCEDE POUR EPAISSIR DES COMPOSITIONS AQUEUSES NOTAMMENT A PH ACIDE, AU MOYEN DE POLYMERES ORGANOPHOSPHATES, ET COMPOSITIONS AQUEUSES OBTENUES**
VERFAHREN ZUM VERDICKEN VON WÄSSRIGEN ZUSAMMENSETZUNGEN, INSBESONDERE MIT SAUREM PH UNTER VERWENDUNG VON ORGANOPHOSPHATPOLYMEREN UND RESULTIERENDE WÄSSRIGE ZUSAMMENSETZUNGEN
METHOD FOR THICKENING AQUEOUS COMPOSITIONS IN PARTICULAR WITH ACIDIC pH, USING ORGANOPHOSPHATE POLYMERS, AND RESULTING AQUEOUS COMPOSITIONS

(30) Priorité: 09.06.2006 FR 0605103
(43) Date de publication de la demande: 25.02.2009
(73) Titulaire: COATEX S.A.S., 69730 Genay (FR)
(72) Inventeur: KENSICHER, Yves, 69620 Theize (FR); SUAU, Jean-Marc, 69480 Lucenay (FR)
(86) Numéro de dépôt international: PCT/IB2007/001515
(87) Numéro de publication internationale: WO 2007/144721

(56) Documents cités:
- EP-A- 1 617 277
- FR-A1- 2 536 758
- FR-A1- 2 637 511

## Description

Un premier objet de l'invention est un procédé d'épaississement d'une composition aqueuse, par introduction dans ladite composition à épaissir d'au moins un polymère, caractérisé en ce que ledit polymère possède un poids moléculaire supèrieur à 80 000g/mole et contient au moins un monomère anionique qui est un monomère organophosphaté.

Un deuxième objet de l'invention consiste en les compositions aqueuses ainsi épaissies et contenant lesdits polymères.

Dans le domaine des formulations aqueuses utilisées dans la cosmétique, telles que les shampoings, les savons, les crèmes, il existe un besoin pour l'homme du métier - formulateur de telles compositions- d'épaissir de tels produits, dans une gamme de pH correspondant à celui de la peau, c'est-à-dire à des valeurs de pH comprises entre 5 et 7, et préférentiellement comprises entre 5 et 6,5, très préférentiellement comprises entre 5,5 et 6.

Afin de résoudre ce problème, l'homme du métier connaît un certain nombre de documents qui peuvent être rangés dans 3 catégories, en fonction des solutions techniques qu'ils proposent : la mise en oeuvre de polymères sous forme de poudres, la technique dite "retour-acide", et enfin la mise en oeuvre de polymères sous forme d'émulsions.

Dans la première catégorie, l'homme du métier connaît le document EP 1 138 703 A1 qui décrit une composition topique cosmétique, dermocosmétique, pharmaceutique ou dermopharmaceutique comprenant de 0,1 % à 10 % en poids d'un polymère, linéaire, branché ou réticulé, à base d'au moins un monomère possédant une fonction acide fort libre telle qu'une fonction sulfonique ou phosphonique, partiellement ou totalement salifiée, copolymérisé avec au moins un monomère choisi soit parmi les esters d'alcools aliphatiques comportant de 8 à 30 atomes de carbone et d'acides monocarboxyliques insaturés, soit parmi les esters d'alcool aliphatiques comportant de 8 à 30 atomes de carbone et d'acides polycarboxyliques insaturés. Le polymère précité est un polymère émulsionnant, sous forme solide, et qui peut être dispersé dans l'eau [0003] ; il permet d'épaissir la composition qui le contient, notamment pour des valeurs de pH supérieures ou égales à 5 [0025]. Il n'existe toutefois aucun exemple qui démontre cette dernière propriété. De plus, le mécanisme d'épaississement de ces polymères n'est pas explicité dans ce document, et il n'existe a fortiori aucune information sur le mécanisme d'épaississement prétendu de ces polymères à des pH inférieurs à 7.

L'homme du métier connaît aussi la technique dite "retour-acide" (selon l'expression anglophone "back-acid"), telle que décrite par exemple dans le document WO 01 / 76 552 qui décrit un procédé permettant d'épaissir un milieu aqueux, ledit procédé consistant à introduire dans ledit milieu aqueux un tensio-actif et un modificateur de rhéologie qui est un copolymère acrylique réticulé et alcali soluble, puis en augmentant ensuite le pH à une valeur supérieure à 5 (préférentiellement 6 et très préférentiellement 6,5) par mise en oeuvre d'un produit alcalin, puis en diminuant le pH (entre 3 et 6 notamment pour des applications cosmétiques) à la valeur souhaitée par ajout d'un composé acide (méthode décrite de la page 27 ligne 25 à la page 28 ligne 21). Un tel copolymère acrylique produit un effet d'épaississement en milieu aqueux lorsque ses groupes acides carboxyliques sont neutralisés, ce qui induit un mécanisme de répulsion ionique conduisant à une augmentation de la viscosité du milieu (page 1, lignes 2 à 8) ; ce mécanisme se distingue notamment du mécanisme d'épaississement, aussi évoqué dans ce document (page 1, lignes 8 à 10) et que connaît bien l'homme du métier. Il consiste en la mise en oeuvre de polymères constitués d'un long squelette hydrophile sur lequel sont greffés des chaînes disposant de groupement hydrophobes qui, une fois introduits dans l'eau, vont conduire à des associations entre les groupements hydrophobes : il y a alors création d'un réseau tridimensionnel qui conduit à une augmentation de la viscosité du milieu.

Enfin, l'homme du métier connaît un certain nombre de documents qui décrivent la mise en oeuvre de polymères en émulsion.

Il connaît notamment le document EP 1 493 774 A2, qui décrit une composition aqueuse pour application topique, contenant un milieu physiologiquement acceptable et au moins un polymère hydrosoluble constitué par un squelette hydrosoluble à base d'acide 2-acrylamido-2-méthylpropane sulfonique, et par des chaînes latérales comprenant au moins un bloc polyoxyéthylène et au moins un bloc polyoxypropylène ou polyoxybutylène. Ces polymères en émulsion permettent d'obtenir un effet thermogélifiant sur une gamme étroite de température, ce qui permet d'avoir une transition fluide / gel plus franche lors de l'application sur la peau de la composition cosmétique les contenant. il est également indiqué que de tels polymères en émulsion sont insensibles au pH, même si aucun exemple n'illustre un effet d'épaississant quelconque pour des valeurs de pH inférieures à 7. L'ensemble de ces propriétés est attribué par les inventeurs [0011 et 0012] à la présence des blocs précités et greffés sur le squelette, à la différence de polymères ayant une répartition statistique des différentes unités monomériques.

L'homme du métier connaît aussi le document EP 0 824 914 B1 qui résout le problème d'épaissir des formulations cosmétiques, aussi bien à pH acides que basiques. La solution qu'il propose consiste en l'utilisation d'un polymère en émulsion comportant au moins un monomère associatif, au moins un monomère d'ester d'alkyle de l'acide acrylique ou méthacrylique, et au moins un monomère choisi dans le groupe constitué des composés hétérocycliques à substituant vinyle ayant au moins un atome d'azote ou de soufre, le méthacrylamide, un méthacrylate de mono- ou di-(alkyle en C1 à C4)amino(alkyle en C1 à C4), un mono- ou di-(alkyle en C1 à C4)amino(alkyle en C1 à C4) méthacrylamide. A la lecture de ce document, il apparaît toutefois que c'est la présence obligatoire d'un monomère cationique aminé qui permet d'épaissir des milieux aqueux à des pH acides : tous les exemples mettent en effet en oeuvre le méthacrylate de diméthylaminoéthyl, comme monomère entrant dans la composition des polymères selon cette invention. Ce document met en oeuvre un mécanisme d'épaississement à pH inférieur à 7 bien connu de l'homme du métier : c'est la présence d'un monomère cationique aminé, qui va s'ioniser à pH acide, et provoquer ainsi le mécanisme d'épaississement.

Un autre exemple de mécanisme d'épaississement à des valeurs de pH inférieures à 7 est décrit dans le document WO 2004 / 024 779 qui vise également à résoudre le problème d'épaissir à des pH acides. La solution qu'il décrit consiste en l'utilisation en émulsion de polymères associatifs de nature cationique, la cationicité étant apportée par un monomère vinylique amino substitué. Dans ce cas, c'est à la fois la présence d'un monomère cationique et les mécanismes d'épaississement associatifs tels que précédemment décrits qui entrent en j eu.

L'homme du métier connaît aussi le document US 4 529 773 B1, qui consiste à épaissir un milieu aqueux par les étapes d'introduction d'une émulsion épaississante alcali soluble mais non hydrosoluble et d'un tensio-actif, de neutralisation du milieu à un pH supérieur à 6,5, et enfin d'acidification du milieu en faisant redescendre le pH à une valeur inférieure à 6,5. il s'agit donc de la combinaison du mécanisme dit retour-acide précédemment décrit, et de la mise en oeuvre d'un polymère sous forme d'émulsion en présence d'un tensio actif. Dans ce document, les inventeurs décrivent l'origine du mécanisme d'épaississement à bas pH de la manière suivante : l'effet épaississant du polymère est "activé" (colonne 3, lignes 41-52) lorsqu'on le neutralise à un pH proche de 7, et cette activation est maintenue même lorsqu'on fait rediminuer le pH de part la présence du tensio-actif.

Enfin, et toujours dans le domaine des polymères en émulsion, l'homme du métier connaît un certain nombre de documents qui décrivent la mise en oeuvre de polymères en émulsion comme agents épaississants, lesdits polymères contenant notamment une fonction phosphonique.

L'homme du métier connaît le document EP 1 371 692 qui décrit des émulsions à base de microlatex auto-réversibles contenant au moins un polymère possédant dans une variante particulière une fonction acide fort qui est la fonction acide sulfonique ou phosphonique.

Il connaît aussi le document FR 2 810 545 qui décrit des émulsions inverses contenant au moins un polymère disposant d'au moins une fonction acide faible et d'au moins une fonction acide fort, ladite fonction acide fort étant selon une variante particulière de cette invention la fonction acide sulfonique ou phosphonique.

Il connaît aussi le document FR 2 856 691 qui décrit une émulsion épaississante contenant de l'eau, un agent émulsionnant et un polyélectrolyte qui peut posséder une fonction acide fort, ladite fonction acide fort pouvant être la fonction acide sulfonique ou phosphonique. Toutefois, aucun des documents précités ne vise à résoudre le problème d'épaississement à des pH inférieurs à 7.

Enfin, l'homme du métier connaît le document WO 03 / 62 288 qui concerne le problème technique de la mise au point de polymères en émulsion, notamment pour applications cosmétiques, offrant des profils rhéologiques particuliers tels que la possibilité de fabriquer des gels à faibles taux de cisaillement, le caractère de gel étant conservé lorsque le taux de cisaillement augmente. Selon un avantage particulier de cette invention, lesdits polymères permettent d'épaissir des formulations aqueuses dans une large gamme de pH, puisqu'à raison de 1 % en poids sec dans l'eau, lesdits polymères conduisent à une viscosité Brookfield™ (à 20 tours / minute) inférieure à 1000 mPa.s et 100 000 mPa.s pour un pH compris entre 3 et 9, et à une viscosité Brookfield™ (à 20 tours / minute) inférieure à 1000 mPa.s pour un pH compris entre 5,5 et 8,5. Pour résoudre ces problèmes techniques, le document WO 03 / 62 288 décrit une solution qui réside dans un polymère associatif et alcali soluble, fabriqué en émulsion, constitué :
a) d'au moins un monomère acide vinylique choisi parmi les monomères vinyliques carboxyliques, ou vinyliques sulfonique ou vinyliques phosphoniques,
b) d'au moins un monomère non-ionique vinylique,
c) d'au moins 2 monomères associatifs terminés par un groupe hydrophobe, les groupes hydrophobes lorsqu'ils sont choisis dans la même classe hydrocarbonée pour les 2 monomères devant alors différer d'au moins 8 atomes de carbone,
d) d'éventuellement au moins un autre monomère choisi parmi un monomère réticulant, un agent de transfert ou leurs mélanges.

Comme le reconnaissent les auteurs de ce document, qui désignent eux-mêmes le polymère en émulsion précité sous le terme de "polymère associatif alcali soluble", le mécanisme d'épaississement est ici à la fois du type associatif et du type alcali soluble (c'est-à-dire qu'il y a activation de l'effet épaississant pour des valeurs de pH acides).

Aussi, poursuivant ses recherches en vue d'épaissir efficacement des compositions. aqueuses, notamment à des pH inférieurs à 7, la Demanderesse a mis au point un procédé d'épaississement d'une composition aqueuse, par introduction dans ladite composition à épaissir d'au moins un polymère, caractérisé en ce que ledit polymère possède un poids moléculaire supèrieur à 80 000g/mole et contient au moins un monomère anionique qui est un monomère organophosphaté.

De manière tout à fait surprenante, la mise en oeuvre d'un tel procédé permet d'obtenir un effet d'épaississement d'une composition aqueuse contenant ledit polymère à monomère organophosphaté, à des valeurs de pH acides, et notamment à des valeurs de pH inférieures à celles auxquelles l'effet d'épaississement apparaît pour la mise en oeuvre de la même quantité d'un polymère épaississant de l'art antérieur.

Dans l'état de la technique précité, la Demanderesse souligne tout d'abord qu'aucun document ne révèle la mise en oeuvre d'un polymère contenant au moins un monomère anionique qui est un monomère organophosphaté, en vue d'épaissir une composition aqueuse. A fortiori, aucun de ces documents ne révèle cette mise en oeuvre pour obtenir un effet d'épaississement à des pH inférieurs à 7.

Sans vouloir être lié à une quelconque théorie, la Demanderesse est de l'opinion que la présence d'un monomère organophosphaté, qui s'ionise facilement à pH inférieur à 7, permet une meilleure solubilisation du polymère et donc un effet d'épaississement notablement marqué à ces valeurs de pH. Aussi, un des mérites de la Demanderesse repose sur le fait qu'elle a su remarquer que la possible ionisation de certains monomères du polymère épaississant, cette ionisation intervenant à des valeurs de pH plus acides que pour des polymères de l'art antérieur tels que des polycarboxylates, et conduisant à une bonne solubilisation du polymère à ces mêmes valeurs de pH, était un mécanisme qui pouvait permettre de déclencher l'effet d'épaississement.

Ce mérite apparaît d'autant plus grand, à la lecture des documents de l'art antérieur précités, que ceux-ci enseignent des mécanismes d'épaississement nombreux et différents (technique de retour-acide, activation d'un polymère en milieu acide, répartition de blocs de chaînons hydrophobes sur une chaîne hydrophile, mécanisme associatif, polymères alcali solubles, polymères à la fois associatifs et alcali solubles), en vue d'épaissir des formulations aqueuses à des pH inférieurs à 7. Rien n'orientait donc a priori l'homme du métier sur le choix d'un de ces mécanismes plutôt qu'un autre.

De plus, la Demanderesse a ensuite su identifier, à travers le choix d'au moins un monomère anionique qui est un monomère organophosphaté, une famille de monomères particulière qui allait donner l'effet désiré, à savoir que lesdits monomères allaient s'ioniser plus facilement que les polymères épaississant de l'art antérieur et ce, à des valeurs de pH inférieures à 7. De la sorte, la solubilisation du polymère contenant ledit monomère est facilitée en milieu acide, et permet le développement d'un mécanisme d'épaississement dont les effets se sont révélés tout à fait surprenants, par rapport à des polymères épaississants de l'art antérieur ne disposant pas de tels monomères organophosphatés.

De plus, la Demanderesse souligne que le procédé selon l'invention permet de mettre en oeuvre comme agents épaississants lesdits polymères contenant au moins un monomère organophosphaté, aussi bien sous la forme de poudre, que sous la forme d'une émulsion, ou que sous la forme d'une solution, ce qui constitue un autre avantage de la présente invention, en terme de possibilités offertes à l'utilisateur.

Enfin, la Demanderesse indique que des polymères contenant un monomère organophosphaté sont déjà connus, et notamment décrits dans le document WO 01 / 74909. Ce document décrit en effet un procédé de synthèse en émulsion d'un polymère constitué d'un monomère polymérisable et d'un surfactant ester polymérisable de formule R₁ - C(O) - R₂ - X, où R₁ désigne un radical vinyle substitué, R₂ désigne un radical polyalkyléne divalent ayant au moins 2 groupements oxyalkylène, et X désigne un groupement phosphate. Ces polymères sont utilisés pour la fabrication de latex, ultérieurement mis en oeuvre dans les peintures. Il n'existe en outre aucun enseignement dans ce document, au sujet d'une éventuelle modification rhéologique que pourraient apporter les polymères mis en oeuvre.

La Demanderesse connaît aussi le document FR 2 536 758 qui décrit des adjuvants pour fluidification de boues de forage aqueuses, dans le but de conserver leurs propriétés rhéologiques dans des conditions de température et de pression extrêmes, caractérisés en ce qu'ils sont des copolymères hydrosolubles résultant de la copolymérisation d'acides éthyléniques, d'acrylamides et d'esters éthyléniques de l'acide phosphorique.

Enfin, la Demanderesse connaît aussi le document FR 2 637 511 qui décrit des agents de compatibilité, de dispersion et de broyage pour suspensions aqueuses pigmentaires formulées à partir de minéraux dont l'un au moins est un sulfate, en vue d'anihiler l'effet viscosifiant induit par la présence dudit sulfate, et possédant (entre autres) la caractéristique de disposer d'une fonction phosphatée ou phosphonée.

En conclusion, au sujet de ces 3 derniers documents : non seulement ils ne divulguent ni ne suggèrent l'utilisation comme agents épaississants de polymères contenant une fonction organophosphatée mais, au contraire, 2 d'entre eux enseignent que des copolymères disposant d'une fonction ester éthylénique de l'acide phosphorique (FR 2 536 758) ou disposant d'une fonction phosphatée ou phosphonée (FR 2 637 511) conduit à des polymères qui ont tendance à diminuer la viscosité du milieu (agents fluidifiants dans le cas du document FR 2 536 758 et agents anihilant l'effet viscosifiant dans le document FR 2 637 511). Or, c'est précisément le contraire que cherche à faire l'homme du métier qui veut résoudre le problème technique posé dans la présente Demande. Par conséquent, ces 3 documents incitaient précisément l'homme du métier à ne pas mettre en oeuvre de polymères possède un poids moléculaire supèrieur à 80 000g/mole et contenant au moins un monomère anionique qui est un monomère organophosphaté, pour épaissir des formulations aqueuses, qui plus est pour épaissir de telles formulations à des pH inférieurs à 7.

Une des caractéristiques distinctives entre les polymères de la présente invention et les polymères mis en oeuvre dans les 2 documents précités réside dans leur poids moléculaire. Dans le document FR 2 637 511, les polymères présentent une viscosité spécifique inférieure à 10 (dans le cas d'un homopolymère de l'acide acrylique, cela signifie que le poids moléculaire est inférieur à 50 000 g/mole) et dans le document FR 2 536 758 ils présentent un poids moléculaire inférieur à 50 000 g/mole. Les polymères de la présente invention possèdent un poids moléculaire supérieur à 80 000 g/mole, préférentiellement supérieur à 100 000 g/mole, très préférentiellement supérieur à 120 000 g/mole (la méthode de mesure est indiquée au début des exemples).

Aussi, un premier objet de l'invention est un procédé d'épaississement d'une composition aqueuse, par introduction dans ladite composition à épaissir d'au moins un polymère, caractérisé en ce que ledit polymère possède un poids moléculaire supèrieur à 80 000g/mole et contient au moins un monomère anionique qui est un monomère organophosphaté.

Ce procédé se caractérise également en ce que le monomère organophosphaté est choisi parmi les molécules de formules : 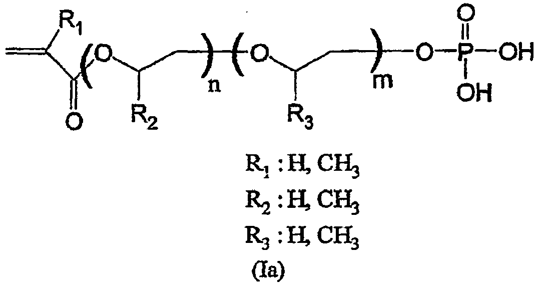 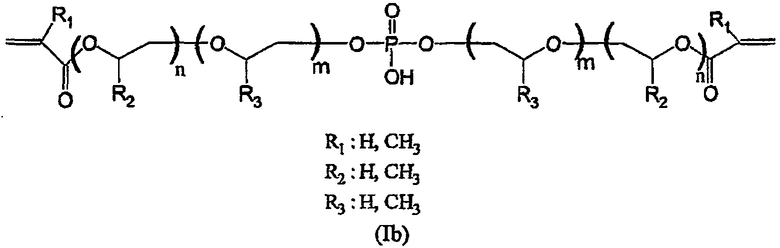 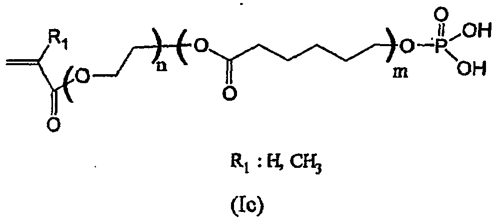 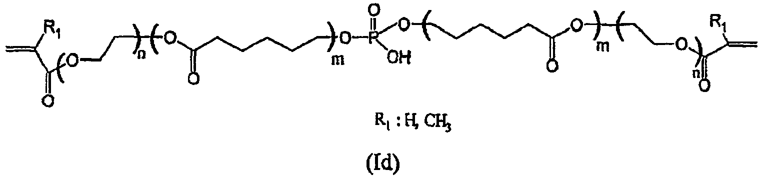 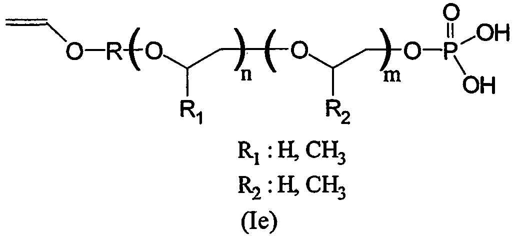 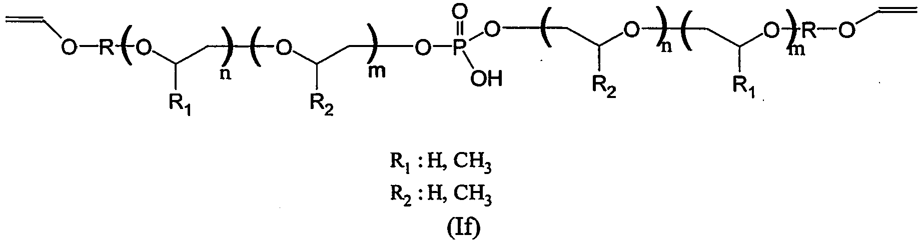 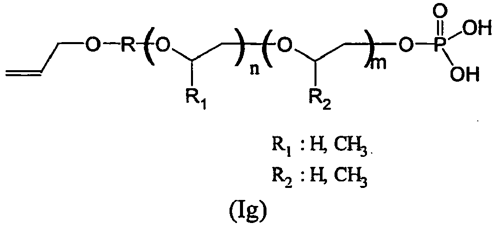 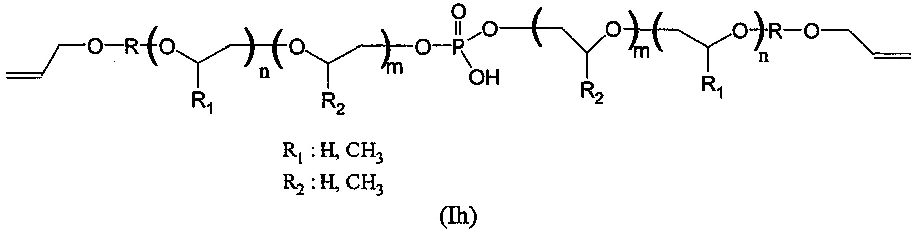 et leurs mélanges, avec n désignant un entier compris entre 1 et 100, préférentiellement entre 1 et 20, m désignant un entier compris entre 0 et 100, préférentiellement entre 0 et 20, et R désignant une chaîne alkyle ayant de 2 à 8 atomes de carbone.

Ce procédé se caractérise également en ce que ladite composition à épaissir possède un pH compris entre 5 et 7, préférentiellement entre 5 et 6,5, très préférentiellement entre 5,5 et 6.

Ce procédé se caractérise également en ce que ledit polymère est introduit dans la composition aqueuse à épaissir sous forme de poudre, et / ou sous forme de dispersion aqueuse, et / ou sous forme de dispersion solvantée, et / ou sous forme de dispersion inverse, et / ou sous forme de solution aqueuse, et / ou sous forme de solution, solvantée. Par dispersion aqueuse, la Demanderesse entend désigner la dispersion dudit polymère sous forme de particules stables, dans une phase continue constituée d'eau (on parlera aussi d'émulsion directe).

Par dispersion solvantée, la Demanderesse entend désigner la dispersion dudit polymère sous forme de particules stables, dans une phase continue constituée d'au moins un solvant.

Par dispersion inverse, la Demanderesse entend désigner un milieu constitué d'une phase contenant ledit polymère et de l'eau, ladite phase étant dispersée dans une phase organique continue (on parlera aussi d'émulsion inverse).

Par solution aqueuse, la Demanderesse entend désigner un milieu constitué dudit polymère dissout dans une phase aqueuse.

Par solution solvantée, la Demanderesse entend désigner un milieu constitué dudit polymère dissout dans une phase solvantée.

Ce procédé se caractérise également en ce qu'il peut aussi mettre en oeuvre la technique retour-acide, c'est-à-dire qu'il comprend les étapes d'introduction dudit polymère dans la composition aqueuse à épaissir, d'introduction d'un composé alcalin permettant d'augmenter la valeur de pH à une valeur supérieure à 5, préférentiellement 6, très préférentiellement 6,5, puis de diminution de la valeur du pH par un composé acide à une valeur inférieure à 7, préférentiellement à 6,5, très préférentiellement inférieure à 5,5.

Ce procédé se caractérise également en ce que ledit polymère contient éventuellement :
a) au moins un autre monomère anionique différent du monomère organophosphaté,
b) et / ou au moins un monomère non-ionique vinylique,
c) et / ou au moins un monomère non-ionique à groupement hydrophobe,
d) et / ou au moins un monomère organofluoré ou organosililé ou leurs mélanges,
e) et / ou au moins un monomère réticulant, c'est-à-dire un monomère ayant au moins 2 liaisons polymérisables, ledit monomère étant différent des monomères organophosphatés de formules (Ib), (Id), (If), et (Ih).

Ce procédé se caractérise également en ce que ledit polymère contient, exprimé en pourcentage en poids de chacun des constituants, de 0,01 à 100 %, préférentiellement de 10 à 100 %, très préférentiellement de 20 à 100 % du monomère organophosphaté, et :
a) de 0 à 90 % d'au moins un autre monomère anionique différent du monomère organophosphaté,
b) de 0 à 50 % d'au moins un monomère non-ionique vinylique,
c) de 0 à 20 % d'au moins un monomère non-ionique à groupement hydrophobe,
d) de 0 à 10 % d'au moins un monomère organofluoré ou organosililé ou leurs mélanges,
e) de 0 à 5 % d'au moins un monomère réticulant, c'est-à-dire un monomère ayant au moins 2 liaisons polymérisables, ledit monomère étant différent des monomères organophosphatés de formules (Ib), (Id), (If), et (Ih),
la somme des pourcentages en poids des monomères constituant ledit polymère étant égale à 100.

Ce procédé se caractérise également en ce que le monomère anionique a) différent du monomère organophosphaté est un monomère à insaturation éthylénique et à fonction carboxylique choisi parmi les monomères à insaturation éthylénique et à fonction monocarboxylique et est alors préférentiellement l'acide acrylique, méthacrylique, crotonique, isocrotonique, cinnamique ou leurs mélanges, ou choisi parmi les hémiesters de diacides et est alors préférentiellement un monoester en C₁ à C₄ des acides maléique ou itaconique, ou leurs mélanges, ou choisi parmi les monomères à insaturation éthylénique et fonction dicarboxylique à l'état acide ou salifié, et préférentiellement parmi l'acide, itaconique, maléique, fumarique, mésaconique ou leurs mélanges ou encore choisi parmi les anhydrides d'acides carboxyliques, et est alors préférentiellement l'anhydride maléique.

Ce procédé se caractérise également en ce que le monomère non-ionique vinylique b) est choisi parmi les esters, les amides ou les nitriles des acides acrylique et méthacrylique, et est alors très préférentiellement choisi parmi les acrylates ou méthacrylates de méthyle, éthyle, butyle, 2-éthyle-hexyle, et leurs mélanges ou est choisi parmi l'acrylonitrile, l'acétate de vinyle, le styrène, le méthylstyrène, le diisobutylène, la vinylpyrrolidone, la vinylcaprolactame et leurs mélanges.

Ce procédé se caractérise également en ce que le monomère non-ionique à groupement hydrophobe c), est un monomère de formule (II) : 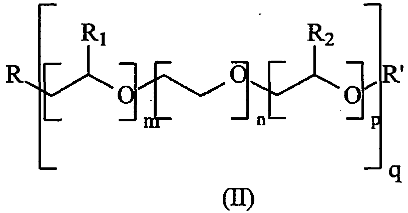 dans laquelle :
- m et p représentent un nombre de motifs d'oxyde d'alkylène inférieur ou égal à 150,
- n représente un nombre de motifs d'oxyde d'éthylène inférieur ou égal à 150,
- q représente un nombre entier au moins égal à 1 et tel que 5 ≤ (m+n+p)q ≤ 150, et préférentiellement tel que 15 ≤ (m+n+p)q ≤ 120,
- R₁ représente l'hydrogène ou le radical méthyle ou éthyle,
- R₂ représente l'hydrogène ou le radical méthyle ou éthyle,
- R représente un radical contenant une fonction insaturée polymérisable, appartenant préférentiellement au groupe des vinyliques ainsi qu'au groupe des esters acrylique, méthacrylique, maléique, itaconique, crotonique, vinylphtalique ainsi qu'au groupe des insaturés uréthannes tels que les acryluréthanne, méthacryluréthanne, α-α' diméthyl-isopropénylbenzyluréthanne, allyluréthanne, de même qu'au groupe des éthers allyliques ou vinyliques substitués ou non, ou encore au groupe des amides ou des imides éthyléniquement insaturées,
- R' représente l'hydrogène ou un radical hydrocarboné ayant 5 à 50 atomes de carbone, et représente préférentiellement un radical hydrocarboné ayant 12 à 50 atomes de carbone et très préférentiellement un radical hydrocarboné ayant 16 à 36 atomes de carbone,

Ce procédé se caractérise également en ce que le monomère organofluoré ou organosililé d), est un monomère de formule (IIIa) ou (IIIb) :
avec formule (IIIa) 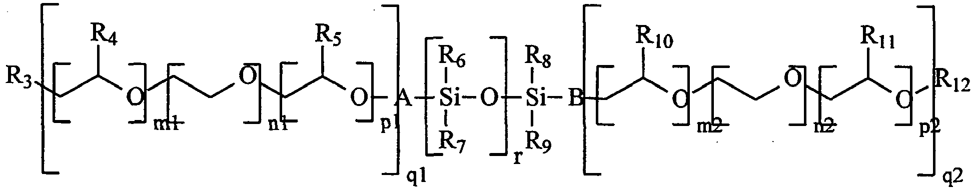 dans laquelle :
   - m₁, p₁, m₂ et p₂ représentent un nombre de motifs d'oxyde d'alkylène inférieur ou égal à 150,
   - n₁ et n₂ représentent un nombre de motifs d'oxyde d'éthylène inférieur ou égal à 150,
   - q₁ et q₂ représentent un nombre entier au moins égal à 1 et tel que 0 ≤ (m₁+n₁+p₁)q₁ ≤ 150 et 0 ≤ (m₂+n₂+p₂)q₂ ≤ 150,
   - r représente un nombre tel que 1 ≤ r ≤ 200,
   - R₃ représente un radical contenant une fonction insaturée polymérisable, appartenant préférentiellement au groupe des vinyliques ainsi qu'au groupe des esters acrylique, méthacrylique, maléique, itaconique, crotonique, vinylphtalique ainsi qu'au groupe des insaturés uréthannes tels que les acryluréthanne, méthacryluréthanne, α-α' diméthyl-isopropénylbenzyluréthanne, allyluréthanne, de même qu'au groupe des éthers allyliques ou vinyliques substitués ou non, ou encore au groupe des amides ou des imides éthyléniquement insaturées,
   - R₄, R₅, R₁₀ et R₁₁, représentent l'hydrogène ou le radical méthyle ou éthyle,
   - R₆, R₇, R₈ et R₉, représentent des groupements linéaires ou ramifiés alkyle, ou aryle, ou alkylaryle, ou arylalkyle ayant 1 à 20 atomes de carbone, ou leur mélange,
   - R₁₂ représente un radical hydrocarboné ayant 1 à 40 atomes de carbone,
   - A et B sont des groupements éventuellement présents, qui représentent alors un radical hydrocarboné ayant 1 à 4 atomes de carbone,
avec formule (IIIb)

   R-A-Si(OB)₃

   dans laquelle :
   - R représente un radical contenant une fonction insaturée polymérisable, appartenant préférentiellement au groupe des vinyliques ainsi qu'au groupe des esters acrylique, méthacrylique, maléique, itaconique, crotonique, vinylphtalique ainsi qu'au groupe des insaturés uréthannes tels que les acryluréthanne, méthacryluréthanne, α-α' diméthyl-isopropénylbenzyluréthanne, allyluréthanne, de même qu'au groupe des éthers allyliques ou vinyliques substitués ou non, ou encore au groupe des amides ou des imides éthyléniquement insaturées,
   - A est un groupement éventuellement présent, qui représente alors un radical hydrocarboné ayant 1 à 4 atomes de carbone,
   - B représente un radical hydrocarboné ayant 1 à 4 atomes de carbone, ou du mélange de plusieurs de ces monomères,

Ce procédé se caractérise également en ce que le monomère réticulant e) est choisi dans le groupe constitué par le diméthacrylate d'éthylène glycol, le triméthylolpropanetriacrylate, l'acrylate d'allyle, les maléates d'allyle, le méthylène-bis-acrylamide, le méthylène-bis-méthacrylamide, le tétrallyloxyéthane, les triallylcyanurates, les éthers allyliques obtenus à partir de polyols tels que le pentaérythritol, le sorbitol, le sucrose, ou choisi parmi les molécules de formule (IV) : 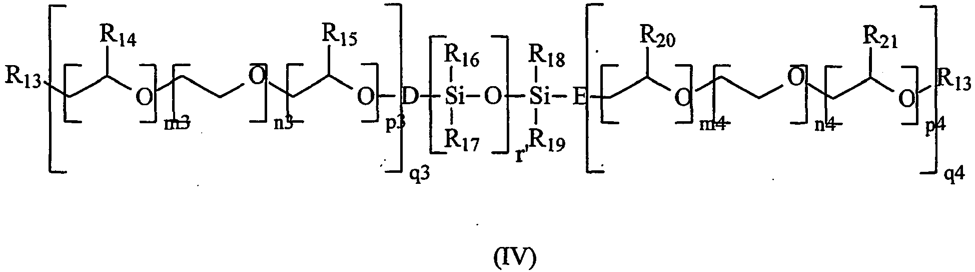 dans laquelle :
- m₃, p₃, m₄ et p₄ représentent un nombre de motifs d'oxyde d'alkylène inférieur ou égal à 150,
- n₃ et n₄ représentent un nombre de motifs d'oxyde d'éthylène inférieur ou égal à 150,
- q₃ et q₄ représentent un nombre entier au moins égal à 1 et tel que 0 ≤ (m₃+n₃+p₃)q₃ ≤ 150 et 0 ≤ (m₄+n₄+p₄)q₄ ≤ 150,
- r' représente un nombre tel que 1 ≤ r' ≤ 200,
- R₁₃ représente un radical contenant une fonction insaturée polymérisable, appartenant préférentiellement au groupe des vinyliques ainsi qu'au groupe des esters acrylique, méthacrylique, maléique, itaconique, crotonique, vinylphtalique ainsi qu'au groupe des insaturés uréthannes tels que les acryluréthanne, méthacryluréthanne, α-α' diméthyl-isopropénylbenzyluréthanne, allyluréthanne, de même qu'au groupe des éthers allyliques ou vinyliques substitués ou non, ou encore au groupe des amides ou des imides éthyléniquement insaturées,
- R₁₄, R₁₅, R₂₀ et R₂₁, représentent l'hydrogène ou le radical méthyle ou éthyle,
- R₁₆, R₁₇, R₁₈ et R₁₉, représentent des groupements linéaires ou ramifiés alkyle, ou aryle, ou alkylaryle, ou arylalkyle ayant 1 à 20 atomes de carbone, ou leur mélange,
- D et E sont des groupements éventuellement présents, qui représentent alors un radical hydrocarboné ayant 1 à 4 atomes de carbone,
ou parmi les mélanges de ces molécules.

Ce procédé se caractérise également en ce que ledit polymère possède un poids moléculaire supérieur à 80 000 g/mole, préférentiellement supérieur à 100 000 g/mole, très préférentiellement supérieur à 120 000 g/mole.

Un autre objet de l'invention réside dans les compositions aqueuses épaissies, caractérisées en ce qu'elles contiennent comme épaississant un polymère contenant au moins un monomère anionique qui est un monomère organophosphaté, ledit polymère ayant un poids moléculaire supérieur à 80 000 g/mole, préférentiellement supérieur à 100 000 g/mole, très préférentiellement supérieur à 120 000 g/mole.

Ces compositions aqueuses se caractérisent également en ce que le monomère organophosphaté est choisi parmi les molécules de formules : 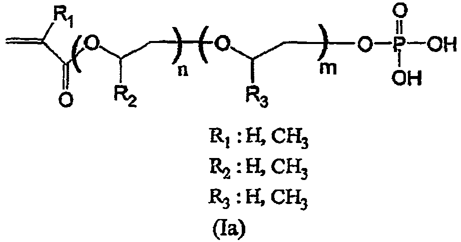 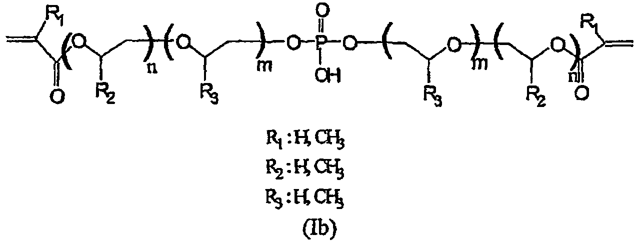 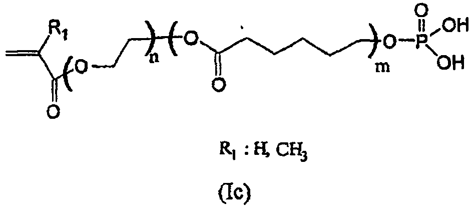 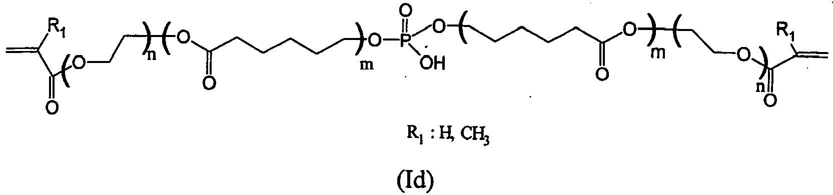 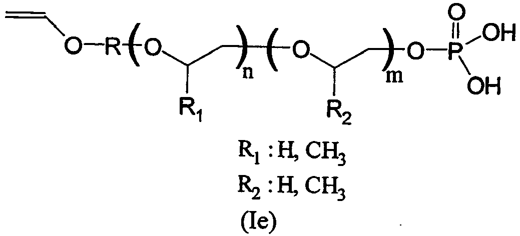 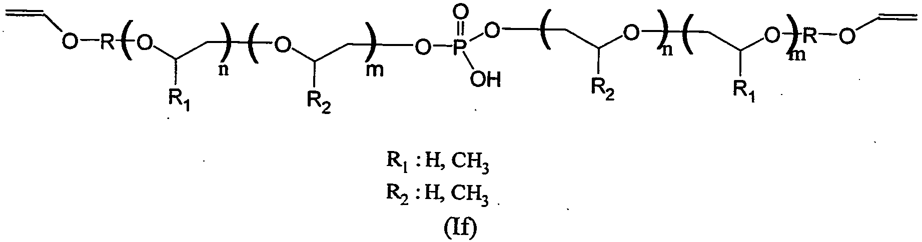 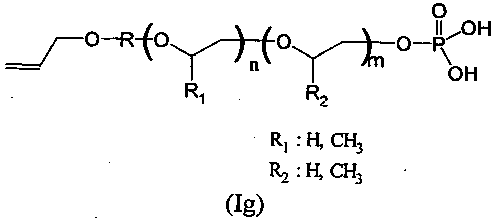 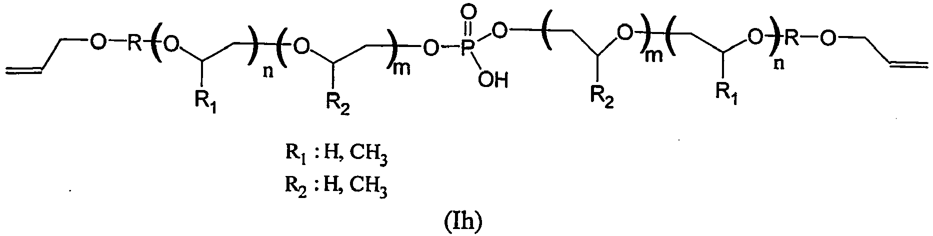 et leurs mélanges, avec n désignant un entier compris entre 1 et 100, préférentiellement entre 1 et 20, m désignant un entier compris entre 0 et 100, préférentiellement entre 0 et 20, et R désignant une chaîne alkyle ayant de 2 à 8 atomes de carbone.

Ces compositions aqueuses se caractérisent également en ce qu'elles possèdent un pH compris entre 5 et 7, préférentiellement entre 5 et 6,5, très préférentiellement entre 5,5 et 6.

Ces compositions aqueuses se caractérisent également en ce que ledit polymère épaississant qu'elles contiennent, contient éventuellement :
a) au moins un autre monomère anionique différent du monomère organophosphaté,
b) et / ou au moins un monomère non-ionique vinylique,
c) et / ou au moins un monomère non-ionique à groupement hydrophobe,
d) et / ou au moins un monomère organofluoré ou organosililé ou leurs mélanges,
e) et / ou au moins un monomère réticulant, c'est-à-dire un monomère ayant au moins 2 liaisons polymérisables, ledit monomère étant différent des monomères organophosphatés de formules (Ib), (Id), (If), et (Ih),

Ces compositions aqueuses se caractérisent également en ce que ledit polymère épaississant qu'elles contiennent contient, exprimé en pourcentage en poids de chacun des constituants, de 0,01 à 100 %, préférentiellement de 10 à 100 %, très préférentiellement de 20 à 100 % du monomère organophosphaté, et :
a) de 0 à 90 % d'au moins un autre monomère anionique différent du monomère organophosphaté,
b) de 0 à 50 % d'au moins un monomère non-ionique vinylique,
c) de 0 à 20 % d'au moins un monomère non-ionique à groupement hydrophobe,
d) de 0 à 10 % d'au moins un monomère organofluoré ou organosililé ou leurs mélanges,
e) de 0 à 5 % d'au moins un monomère réticulant, c'est-à-dire un monomère ayant au moins 2 liaisons polymérisables, ledit monomère étant différent des monomères organophosphatés de formules (Ib), (Id), (If), et (Ih),
la somme des pourcentages en poids des monomères constituant ledit polymère étant égale à 100.

Ces compositions aqueuses se caractérisent également en ce que le monomère anionique a) du polymère épaississant qu'elles contiennent, est un monomère à insaturation éthylénique et à fonction carboxylique choisi parmi les monomères à insaturation éthylénique et à fonction monocarboxylique et est alors préférentiellement l'acide acrylique, méthacrylique, crotonique, isocrotonique, cinnamique ou leurs mélanges, ou choisi parmi les hémiesters de diacides et est alors préférentiellement un monoester en C₁ à C₄ des acides maléique ou itaconique, ou leurs mélanges, ou choisi parmi les monomères à insaturation éthylénique et fonction dicarboxylique à l'état acide ou salifié, et préférentiellement parmi l'acide, itaconique, maléique, fumarique, mésaconique ou leurs mélanges ou encore choisi parmi les anhydrides d'acides carboxyliques, et est alors préférentiellement l'anhydride maléique.

Ces compositions aqueuses se caractérisent également en ce que le monomère non-ionique vinylique b) du polymère épaississant qu'elles contiennent, est choisi parmi les esters, les amides ou les nitriles des acides acrylique et méthacrylique, et est alors très préférentiellement choisi parmi les acrylates ou méthacrylates de méthyle, éthyle, butyle, 2-éthyle-hexyle, et leurs mélanges ou est choisi parmi l'acrylonitrile, l'acétate de vinyle, le styrène, le méthylstyrène, le diisobutylène, la vinylpyrrolidone, la vinylcaprolactame et leurs mélanges.

Ces compositions aqueuses se caractérisent également en ce que le monomère non-ionique à groupement hydrophobe c) du polymère épaississant qu'elles contiennent, est un monomère de formule (II) : 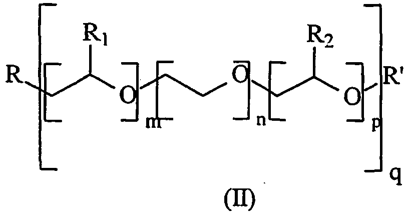 dans laquelle :
- m et p représentent un nombre de motifs d'oxyde d'alkylène inférieur ou égal à 150,
- n représente un nombre de motifs d'oxyde d'éthylène inférieur ou égal à 150,
- q représente un nombre entier au moins égal à 1 et tel que 5 ≤ (m+n+p)q ≤ 150, et préférentiellement tel que 15 ≤ (m+n+p)q ≤ 120,
- R₁ représente l'hydrogène ou le radical méthyle ou éthyle,
- R₂ représente l'hydrogène ou le radical méthyle ou éthyle,
- R représente un radical contenant une fonction insaturée polymérisable, appartenant préférentiellement au groupe des vinyliques ainsi qu'au groupe des esters acrylique, méthacrylique, maléique, itaconique, crotonique, vinylphtalique ainsi qu'au groupe des insaturés uréthannes tels que les acryluréthanne, méthacryluréthanne, α-α' diméthyl-isopropénylbenzyluréthanne, allyluréthanne, de même qu'au groupe des éthers allyliques ou vinyliques substitués ou non, ou encore au groupe des amides ou des imides éthyléniquement insaturées,
- R' représente l'hydrogène ou un radical hydrocarboné ayant 5 à 50 atomes de carbone, et représente préférentiellement un radical hydrocarboné ayant 12 à 50 atomes de carbone et très préférentiellement un radical hydrocarboné ayant 16 à 36 atomes de carbone,

Ces compositions aqueuses se caractérisent également en ce que le monomère organofluoré ou organosililé d) du polymère épaississant qu'elles contiennent, est un monomère de formule (IIIa) ou (IIIb) :
avec formule (IIIa) 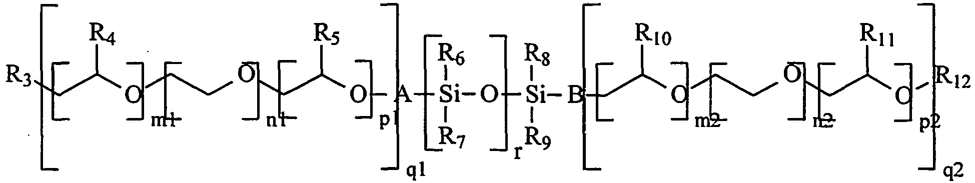 dans laquelle :
   - m₁, p₁, m₂ et p₂ représentent un nombre de motifs d'oxyde d'alkylène inférieur ou égal à 150,
   - n₁ et n₂ représentent un nombre de motifs d'oxyde d'éthylène inférieur ou égal à 150,
   - q₁ et q₂ représentent un nombre entier au moins égal à 1 et tel que 0 ≤ (m₁+n₁+p₁)q₁ ≤ 150 et 0 ≤ (m₂+n₂+p₂)q₂ ≤ 150,
   - r représente un nombre tel que 1 ≤ r ≤ 200,
   - R₃ représente un radical contenant une fonction insaturée polymérisable, - appartenant préférentiellement au groupe des vinyliques ainsi qu'au groupe des esters acrylique, méthacrylique, maléique, itaconique, crotonique, vinylphtalique ainsi qu'au groupe des insaturés uréthannes tels que les acryluréthanne, méthacryluréthanne, α-α' diméthyl-isopropénylbenzyluréthanne, allyluréthanne, de même qu'au groupe des éthers allyliques ou vinyliques substitués ou non, ou encore au groupe des amides ou des imides éthyléniquement insaturées,
   - R₄, R₅, R₁₀ et R₁₁, représentent l'hydrogène ou le radical méthyle ou éthyle,
   - R₆, R₇, R₈ et R₉, représentent des groupements linéaires ou ramifiés alkyle, ou aryle, ou alkylaryle, ou arylalkyle ayant 1 à 20 atomes de carbone, ou leur mélange,
   - R₁₂ représente un radical hydrocarboné ayant 1 à 40 atomes de carbone,
   - A et B sont des groupements éventuellement présents, qui représentent alors
      un radical hydrocarboné ayant 1 à 4 atomes de carbone,
avec formule (IIIb)

   R-A-Si(OB)₃

   dans laquelle :
   - R représente un radical contenant une fonction insaturée polymérisable, appartenant préférentiellement au groupe des vinyliques ainsi qu'au groupe des esters acrylique, méthacrylique, maléique, itaconique, crotonique, vinylphtalique ainsi qu'au groupe des insaturés uréthannes tels que les acryluréthanne, méthacryluréthanne, α-α' diméthyl-isopropénylbenzyluréthanne, allyluréthanne, de même qu'au groupe des éthers allyliques ou vinyliques substitués ou non, ou encore au groupe des amides ou des imides éthyléniquement insaturées,
   - A est un groupement éventuellement présent, qui représente alors un radical hydrocarboné ayant 1 à 4 atomes de carbone,
   - B représente un radical hydrocarboné ayant 1 à 4 atomes de carbone, ou du mélange de plusieurs de ces monomères,
Ces compositions aqueuses se caractérisent également en ce que le monomère réticulant e) du polymère épaississant qu'elles contiennent, est choisi dans le groupe constitué par le diméthacrylate d'éthylène glycol, le triméthylolpropanetriacrylate, l'acrylate d'allyle, les maléates d'allyle, le méthylène-bis-acrylamide, le méthylène-bis-méthacrylamide, le tétrallyloxyéthane, les triallylcyanurates, les éthers allyliques obtenus à partir de polyols tels que le pentaérythritol, le sorbitol, le sucrose, ou choisi parmi les molécules de formule (IV) : 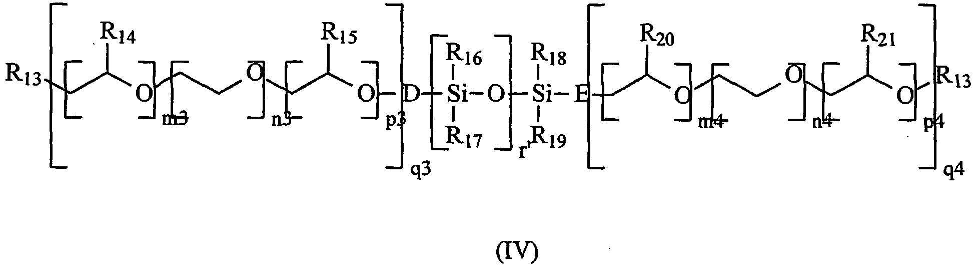 dans laquelle :
- m₃, p₃, m₄ et p₄ représentent un nombre de motifs d'oxyde d'alkylène inférieur ou égal à 150,
- n₃ et n₄ représentent un nombre de motifs d'oxyde d'éthylène inférieur ou égal à 150,
- q₃ et q₄ représentent un nombre entier au moins égal à 1 et tel que 0 ≤ (m₃+n₃+p₃)q₃ ≤ 150 et 0 ≤ (m₄+n₄+p₄)q₄ ≤ 150,
- r' représente un nombre tel que 1 ≤ r' ≤ 200,
- R₁₃ représente un radical contenant une fonction insaturée polymérisable, appartenant préférentiellement au groupe des vinyliques ainsi qu'au groupe

des esters acrylique, méthacrylique, maléique, itaconique, crotonique, vinylphtalique ainsi qu'au groupe des insaturés uréthannes tels que les acryluréthanne, méthacryluréthanne, α-α' diméthyl-isopropénylbenzyluréthanne, allyluréthanne, de même qu'au groupe des éthers allyliques ou vinyliques substitués ou non, ou encore au groupe des amides ou des imides éthyléniquement insaturées,
- R₁₄, R₁₅, R₂₀ et R₂₁, représentent l'hydrogène ou le radical méthyle ou éthyle,
- R₁₆, R₁₇, R₁₈ et R₁₉, représentent des groupements linéaires ou ramifiés alkyle, ou aryle, ou alkylaryle, ou arylalkyle ayant 1 à 20 atomes de carbone, ou leur mélange,
- D et E sont des groupements éventuellement présents, qui représentent alors un radical hydrocarboné ayant 1 à 4 atomes de carbone,
ou parmi les mélanges de ces molécules.

Ces compositions aqueuses sont aussi caractérisées en ce qu'elles sont des compositions cosmétiques, pharmaceutiques, des compositions à base de liants hydrauliques et sont alors préférentiellement des bétons, des ciments, des mortiers, des coulis, des laitiers, des compositions détergentes, des sauces de couchage papetières, des peintures.

Les exemples suivants illustrent l'invention sans toutefois en limiter la portée.

### EXEMPLES

Dans tous les exemples, la demanderesse indique que le poids moléculaire des polymères est déterminé selon la méthode suivante :
Il s'agit d'une méthode d'analyse par Chromatographie d'Exclusion Stérique (CES).
L'éluant de la CES est le tétrahydrofurane.

Le débit de produit à analyser est de 0,8 mL/min.

Le produit à analyser est constitué de 0,4 % en poids sec de polymère à tester dans la phase mobile, incluant aussi 0,2 % en poids sec de diméthyl formamide.

La chaîne de CES contient une pompe isocratique (Waters™ 515), un four contenant une précolonne de type "Guard Column Styragel Waters™", deux colonnes linéaires de 7,8 mm de diamètre interne et 30 cm de longueur de type "Styragel^{™} Waters HR4E" et un détecteur réfractométrique de type RI Waters™ 410.

La température des colonnes et du détecteur est fixée à 35 °C.

Le logiciel de détection et de traitement du chromatogramme est le logiciel PSS win GPC scientific V 4.02.

La CES est étalonnée par une série de 5 étalons de poly(acrylate) de sodium fournis par Polymer Standards Service™.

La colonne est étalonnée au moyen d'étalons de polyéthylène glycol fournis par Polymer Standards Service (PSS), et de poids moléculaires compris entre 3 000 et 12 000 g/mole.

### Exemple 1

Cet exemple illustre le procédé selon l'invention, en vue d'épaissir une composition aqueuse qui est une formulation cosmétique de crème de nuit, par introduction dans ladite composition à épaissir d'un polymère contenant un monomère organophosphaté.

Cet exemple illustre également le procédé selon l'invention dans lequel ledit polymère est mis en oeuvre sous forme de dispersion aqueuse.

Cet exemple illustre également le procédé selon l'invention dans lequel le phénomène d'épaississement intervient à un pH acide compris entre 5,5 et 5,6.

Enfin, cet exemple illustre aussi la composition aqueuse selon l'invention qui est une crème de nuit, et qui contient ledit polymère.

### Essai n°1

Pour l'essai n° 1 illustrant l'invention, on réalise une formulation de crème de nuit dont la composition est donnée dans le tableau 1.

Cet essai illustre l'invention et met en oeuvre une dispersion aqueuse constituée de :
- 80 % en poids d'eau,
- 20 % en poids d'un polymère de poids moléculaire égal à 135 000 g/mole,
ledit polymère étant constitué de, exprimé en pourcentage en poids des monomères :
35 % d'un mélange de monomères de formule (Ia) et de formule (Ib) dans lesquelles R₁ = CH₃, R₂ = H, n = 1, m = 0
a) 30 % d'acide méthacrylique,
b) 24,8 % d'acrylate d'éthyle,
c) 10,2 % d'un monomère de formule (II) avec :
R désignant le groupement méthacrylate,
R₁ et R₂ désignant l'hydrogène,
n+m+p=25
R' désignant le radical hydrocarboné ayant 22 atomes de carbone.

**Tableau 1**

| **Composition de la crème pour 100 g de produit fini** | | |
|---|---|---|
| **Ordre d'introduction** | **Noms INCI (Internnational Nomenclature of Cosmetic Ingredients) des composés** | **Masse (g)** |
| A | Propylene Glycol Dipelargonate | 6,00 |
| A | Sucrose Distearate | 3,50 |
| A | Isostearyl Isostearate | 6,00 |
| A | Cyclomethicone | 4,00 |
| A | Hydrogenated Polydecene | 4,00 |
| A | Phenoxyethanol (and) Methylparaben (and) Ethylparaben (and) Butylparaben (and) Propylparaben (and) Isobutylparaben | 0,50 |
| A | Sucrose Stearate | 1,50 |
| B | Eau déminéralisée | 71,00 |
| B | Dispersion aqueuse contenant le polymère organophosphaté | 3,30 |
| C | Hydroxyde de sodium 10% (qsp pH 5,6) | 0,20 |
| TOTAL | | 100,00 |

Les composés correspondant à l'ordre d'introduction [A] sont pesés puis fondus et homogénéisés à 70°C.

Sous agitation, les composés correspondant à l'ordre d'introduction [B] sont alors ajoutés au mélange qui est ensuite émulsionné au moyen d'une turbine à grande vitesse (6 000 tours par minute).

Le pH de l'émulsion est ensuite élevé à une valeur de 5,5 - 5,6 au moyen d'hydroxyde de sodium [C], la vitesse d'agitation étant augmentée jusqu'à une valeur de 10 000 tours par minute afin de compenser l'augmentation de la viscosité.

### Essai n° 2

Cet essai correspond à un témoin, pour lequel on a réalisé la même composition de crème de nuit que celle indiquée pour l'essai n° 1, à la différence que ladite composition ne contient pas le polymère avec un monomère organophosphaté (on a introduit la même quantité d'eau déminéralisée à la place).

Les compositions correspondant aux essais n° 1 et 2 ont été stockées dans un flacon fermé pendant 24 heures à 25°C. Passé ce délai, la viscosité de la formulation est mesurée au moyen d'un viscosimètre Brookfield™ à des vitesses de 2,5, 5, 10, 20, 50 et 100 tours par minute, les résultats obtenus étant indiqués dans le tableau 2.

**Tableau 2**

| **Mesure de la viscosité Brookfield™ (en mPa.s) de la crème** | | |
|---|---|---|
| **Vitesse de mesure** | **Essai n° 2** (témoin) | **Essai n° 1** (invention) |
| 2,5 tours par minute | 26000 | 81000 |
| 5 tours par minute | 14200 | 44500 |
| 10 tours par minute | 8000 | 24250 |
| 20 tours par minute | 4500 | 13250 |
| 50 tours par minute | 2180 | 6150 |
| 100 tours par minute | 1260 | 3650 |

Les résultats du tableau 2 démontrent l'effet épaississant très marqué obtenu à un pH compris entre 5,5 et 5,6 dans le cas de l'invention, c'est-à-dire par la mise en oeuvre du polymère contenant le monomère organophosphaté.

### Exemple 2

Cet exemple illustre le procédé selon l'invention, en vue d'épaissir une composition aqueuse qui est une émulsion cosmétique de crème de soin pour le corps, par introduction dans ladite composition à épaissir d'un polymère contenant un monomère organophosphaté.

Cet exemple illustre également le procédé selon l'invention dans lequel ledit polymère est mis en oeuvre sous forme de dispersion aqueuse.

Cet exemple illustre également le procédé selon l'invention dans lequel le phénomène d'épaississement intervient à un pH acide compris entre 5,5 et 5,6.

Enfin, cet exemple illustre aussi la composition aqueuse selon l'invention qui est une émulsion cosmétique de crème de soin pour le corps, et qui contient ledit polymère. Pour les essais n° 3 à 11, on réalise une formulation de crème de soin pour le corps dont la composition est donnée dans le tableau 3.

### Essai n° 3

L'essai n° 3 illustre l'art antérieur et met en oeuvre un polymère constitué d'acrylate d'éthyle, d'acide méthacrylique, et de diméthacrylate d'éthylène glycol.

### Essai n° 4

Cet essai illustre l'invention et met en oeuvre une dispersion aqueuse constituée de :
- 80 % en poids d'eau,
- 20 % en poids d'un polymère de poids moléculaire égal à 125 000 g/mole,
   ledit polymère étant constitué de, exprimé en pourcentage en poids des monomères :
   35 % d'un mélange de monomères de formule (Ia) et de formule (Ib) dans lesquelles R₁ = CH₃, R₂ = CH₃, R₃ = H, n = 6, m = 3,
      a) 30,0 % d'acide méthacrylique,
      b) 24,8 % d'acrylate d'éthyle,
      c) 10,2 % d'un monomère de formule (II) avec :
         R désignant le groupement méthacrylate,
         R₁ et R₂ désignant l'hydrogène,
         n + m + p = 25
         R' désignant le radical hydrocarboné ayant 22 atomes de carbone.

### Essai n° 5

Cet essai illustre l'invention et met en oeuvre une dispersion aqueuse constituée de :
- 80 % en poids d'eau,
- 20 % en poids d'un polymère de poids moléculaire égal à 124 000 g/mole,
   ledit polymère étant constitué de, exprimé en pourcentage en poids des monomères :
   35 % d'un mélange de formule (Ia) et (Ib) dans lesquelles R₁ = CH₃, R₂ = H, R₃ = H,n+m=100,
      a) 30,0 % d'acide méthacrylique,
      b) 24,8 % d'acrylate d'éthyle,
      c) 10,2 % d'un monomère de formule (II) avec :
         R désignant le groupement méthacrylate,
         R₁ et R₂ désignant l'hydrogène,
         n+m+p=25
         R' désignant le radical hydrocarboné ayant 22 atomes de carbone.

### Essai n° 6

Cet essai illustre l'invention et met en oeuvre une dispersion aqueuse constituée de :
- 80 % en poids d'eau,
- 20 % en poids d'un polymère de poids moléculaire égal à 108 000 g/mole,
   ledit polymère étant constitué de, exprimé en pourcentage en poids des monomères :
   35 % d'un mélange de monomères de formule (Ic) et de formule (Id) dans lesquelles R₁ = CH₃, n = 1, m = 2,
      a) 30,0 % d'acide méthacrylique,
      b) 24,8 % d'acrylate d'éthyle,
      c) 10,2 % d'un monomère de formule (II) avec :
         R désignant le groupement méthacrylate,
         R₁ et R₂ désignant l'hydrogène,
         n+m+p=25
         R' désignant le radical hydrocarboné ayant 22 atomes de carbone.

### Essai n° 7

Cet essai illustre l'invention et met en oeuvre une dispersion aqueuse constituée de :
- 80 % en poids d'eau,
- 20 % en poids d'un polymère de poids moléculaire égal à 150 000 g/mole,
   ledit polymère étant constitué de, exprimé en pourcentage en poids des monomères :
   35 % d'un mélange de monomères de formule (Ia) et de formule (Ib) dans lesquelles R₁ = CH₃, R₂ = H, n = 1, m = 0
      a) 39 % d'acide méthacrylique,
      b) 20 % d'acrylate d'éthyle,
      c) 6 % d'un monomère de formule (II) avec :
         R désignant le groupement méthacrylate,
         R₁ et R₂ désignant l'hydrogène,
         n + m + p = 25
         R' désignant le radical hydrocarboné ayant 22 atomes de carbone.

### Essai n° 8

Cet essai illustre l'invention et met en oeuvre une dispersion aqueuse constituée de :
- 80 % en poids d'eau,
- 20 % en poids d'un polymère de poids moléculaire égal à 117 000 g/mole,
   ledit polymère étant constitué de, exprimé en pourcentage en poids des monomères :
   35 % d'un mélange de monomères de formule (Ia) et de formule (Ib) dans lesquelles R₁ = CH₃, R₂ = H, n = 1, m = 0
      a) 20 % d'acide méthacrylique,
      b) 39 % d'acrylate d'éthyle,
      c) 6 % d'un monomère de formule (II) avec :
         R désignant le groupement méthacrylate,
         R₁ et R₂ désignant l'hydrogène,
         n + m + p = 25
         R' désignant le radical hydrocarboné ayant 22 atomes de carbone.

### Essai n° 9

Cet essai illustre l'invention et met en oeuvre une dispersion aqueuse constituée de :
- 80 % en poids d'eau,
- 20 % en poids d'un polymère de poids moléculaire égal à 145 000 g/mole, ledit polymère étant constitué de, exprimé en pourcentage en poids des monomères :
   35 % d'un mélange de monomères de formule (Ia) et de formule (Ib) dans lesquelles R₁ = CH₃, R₂ = H, n = 1, m = 0
      a) 36 % d'acide méthacrylique,
      b) 20 % d'acrylate d'éthyle,
      c) 9 % d'un monomère de formule (II) avec :
         R désignant le groupement méthacrylate,
         R₁ et R₂ désignant l'hydrogène,
         n + m + p = 25
         R' désignant le radical hydrocarboné ayant 22 atomes de carbone.

### Essai n° 10

Cet essai illustre l'invention et met en oeuvre une dispersion aqueuse constituée de :
- 80 % en poids d'eau,
- 20 % en poids d'un polymère de poids moléculaire égal à 142 500 g/mole,
   ledit polymère étant constitué de, exprimé en pourcentage en poids des monomères :
   35 % d'un mélange de monomères de formule (Ia) et de formule (Ib) dans lesquelles R₁ = CH₃, R₂ = H, n = 1, m = 0
      a) 38 % d'acide méthacrylique,
      b) 20 % d'acrylate d'éthyle,
      c) 7 % d'un monomère de formule (II) avec :
         R désignant le groupement méthacrylate,
         R₁ et R₂ désignant l'hydrogène,
         n + m + p = 25
         R' désignant le radical hydrocarboné ayant 22 atomes de carbone.

### Essai n° 11

Cet essai illustre l'invention et met en oeuvre une dispersion aqueuse constituée de :
- 80 % en poids d'eau,
- 20 % en poids d'un polymère de poids moléculaire égal à 105 500 g/mole,
ledit polymère étant constitué de, exprimé en pourcentage en poids des monomères :
35 % d'un mélange de monomères de formule (Ia) et de formule (Ib) dans lesquelles R₁ = CH₃, R₂ = H, n = 1, m = 0
a) 30,7 % d'acide méthacrylique,
b) 25,3 % d'acrylate d'éthyle,
c) 9 % d'un monomère de formule (II) avec :
R désignant le groupement méthacrylate,
R₁ et R₂ désignant l'hydrogène,
n+m+p=25
R' désignant le radical hydrocarboné ayant 22 atomes de carbone.

**Tableau 3**

| **Composition de l'émulsion pour 100 g de produit fini** | | |
|---|---|---|
| Ordre d'introduction | **Noms INCI des composés** | **Masse (g)** |
| A | Cetyl Alcohol (and) Glyceryl Stearate (and) PEG-75 Stearate (and) Ceteth-20 (and) Steareth-20 | 3,00 |
| A | Octyl Dodecyl Myristate | 3,00 |
| A | Caprylic/Capric Triglyceride | 3,00 |
| A | Dimethicone | 4,00 |
| A | Cyclomethicone | 5,00 |
| A | Isopropyl Palmitate | 3,00 |
| A | Phenoxyethanol (and) Methylparaben (and) Ethylparaben (and) Butylparaben (and) Propylparaben (and) Isobutylparaben | 0,50 |
| B | Eau déminéralisée | 69,84 |
| B | Dispersion aqueuse contenant le polymère épaississant | 3,30 |
| C | Hydroxyde de sodium 10% (qsp pH 5,6) | 0,16 |
| D | Parfum | 0,20 |
| E | Hydrolyzed Soy Protein | 5,00 |
| TOTAL | | 100,00 |

Les composés correspondant à l'ordre d'introduction [A] sont pesés puis fondus et homogénéisés à 70°C.

Sous agitation, les composés correspondant à l'ordre d'introduction [B] sont alors ajoutés au mélange qui est ensuite émulsionné au moyen d'une turbine à grande vitesse (6 000 tours par minute).

Le pH de l'émulsion est ensuite élevé à une valeur de 5,5 - 5,6 au moyen d'hydroxyde de sodium [C], la vitesse d'agitation étant augmentée jusqu'à une valeur de 10 000 tours par minute afin de compenser l'augmentation de la viscosité.

Tout en maintenant l'agitation, les ingrédients restants [D] et [E] sont ajoutés et homogénéisés.

### Essai n° 12

Cet essai correspond à un témoin réalisé sans épaississant. La quantité de dispersion aqueuse contenant l'épaississant a été remplacée dans la formulation par la même quantité d'eau déminéralisée.

Les formulations correspondant aux essais n° 3 à 12 sont stockées dans un flacon fermé pendant 24 heures à 25°C. Passé ce délai, la viscosité de la formulation est mesurée au moyen d'un viscosimètre Brookfield™ à des vitesses de 2,5, 5, 10, 20, 50 et 100 tours par minute les résultats obtenus étant indiqués dans le tableau 4.

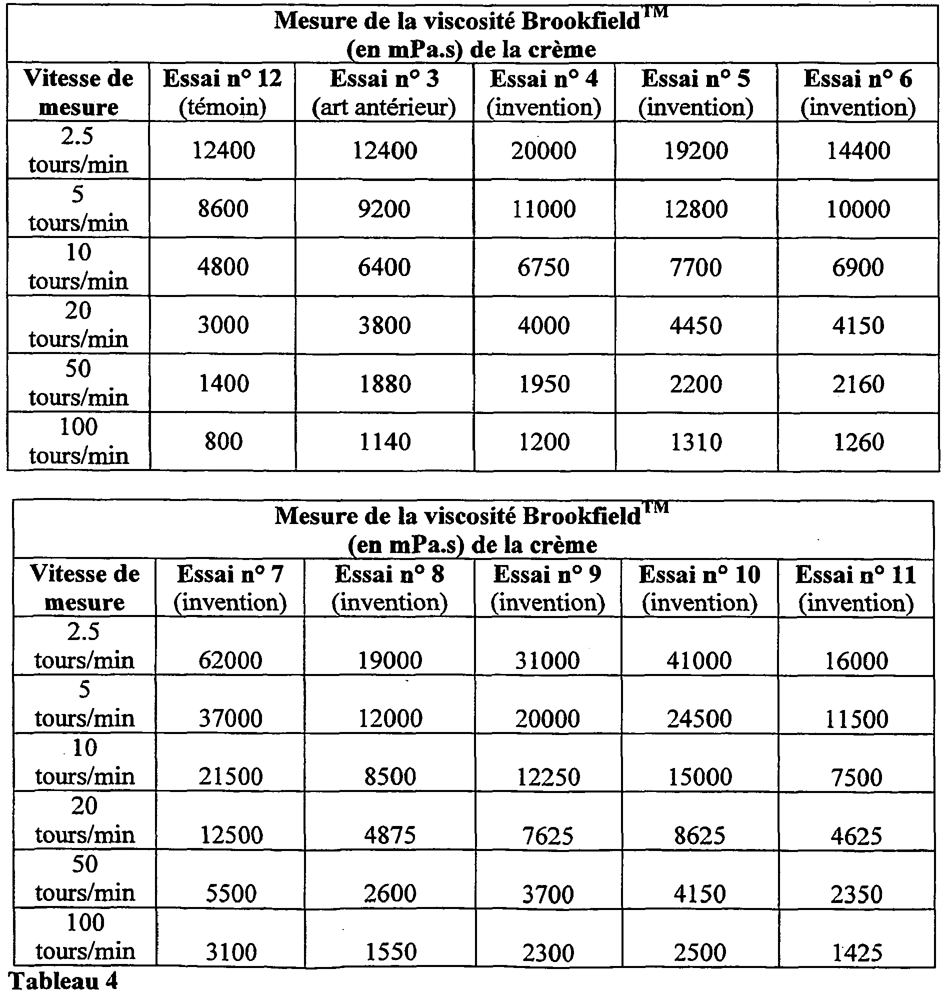

Les résultats du tableau 4 démontrent que, pour un pH compris entre 5,5 et 5,6, l'effet épaississant est plus important dans le cas de l'invention, que dans celui du témoin et de l'art antérieur et ce, quelle que soit la vitesse de mesure de la viscosité Brookfield™.

### Exemple 3

Cet exemple illustre le procédé selon l'invention, en vue d'épaissir une composition aqueuse qui est une formulation cosmétique de lait fluide pour le corps, par introduction dans ladite composition à épaissir d'un polymère contenant un monomère organophosphaté.

Cet exemple illustre également le procédé selon l'invention dans lequel ledit polymère est mis en oeuvre sous forme de dispersion aqueuse.

Cet exemple illustre également le procédé selon l'invention dans lequel le phénomène d'épaississement intervient à un pH acide compris entre 6,2 et 6,3.

Enfin, cet exemple illustre aussi la composition aqueuse selon l'invention qui est une formulation cosmétique de lait fluide pour le corps, et qui contient ledit polymère.

### Essai n° 13

Pour l'essai n° 13 illustrant l'invention, on réalise une formulation de lait fluide pour le corps dont la composition est donnée dans le tableau 5.

Cet essai illustre l'invention et met en oeuvre une dispersion aqueuse constituée de :
- 80 % en poids d'eau,
- 20 % en poids d'un polymère de poids moléculaire égal à 124 000 g/mole,
ledit polymère étant constitué de, exprimé en pourcentage en poids des monomères :
35 % d'un mélange de formule (Ia) et (Ib) dans lesquelles R₁ = CH₃, R₂ = H, R₃ = H, n + m = 100,
a) 30,0 % d'acide méthacrylique,
b) 24,8 % d'acrylate d'éthyle,
c) 10,2 % d'un monomère de formule (II) avec :
R désignant le groupement méthacrylate,
R₁ et R₂ désignant l'hydrogène,
n + m + p = 25
R' désignant le radical hydrocarboné ayant 22 atomes de carbone.

**Tableau 5**

| **Composition du lait pour 100 g de produit fini** | | |
|---|---|---|
| **Ordre d'introduction** | **Noms INCI des composés** | **Masse (g)** |
| A | Propylene Glycol Dipelargonate | 6,00 |
| A | Hydrogenated Polydecene | 5,00 |
| A | Caprylic/Capric Triglyceride | 3,00 |
| A | Tocopheryl Acetate | 0,50 |
| A | Phenoxyethanol (and) Methylparaben (and) Ethylparaben (and) Butylparaben (and) Propylparaben (and) Isobutylparaben | 0,50 |
| B | Propylene Glycol Dipelargonate | 1,00 |
| B | Dispersion aqueuse contenant le polymère épaississant | 3,38 |
| C | Eau déminéralisée | 80,32 |
| D | Hydroxyle de sodium 10% (qsp pH 5,6) | 0,30 |
| TOTAL | | 100,00 |

Les composés correspondant à l'ordre d'introduction [A] sont pesés puis homogénéisés.

Les composés correspondant à l'ordre d'introduction [B] sont pesés, homogénéisés puis mélangés au composé correspondant à l'ordre d'introduction [C].

Sous agitation, le mélange des composés correspondant aux ordres d'introduction [B] et [C] est introduit dans le mélange correspondant à l'ordre d'introduction [A], le tout étant ensuite émulsionné au moyen d'une turbine à grande vitesse (6 000 tours par minute).

L'agitation étant maintenue à 6 000 tours par minute

Le pH du lait est ensuite élevé à une valeur de 6,2 - 6,3 au moyen d'hydroxyde de sodium [D].

### Essai n° 14

Cet essai correspond à un témoin, pour lequel on a réalisé la même composition de lait fluide pour le corps que celle indiquée pour l'essai n° 13, à la différence que ladite composition ne contient pas le polymère avec un monomère organophosphaté (on a introduit la même quantité d'eau déminéralisée à la place de la dispersion aqueuse contenant ledit polymère).

Les compositions correspondant aux essais n° 13 et 14 ont été stockées dans un flacon fermé pendant 24 heures à 25°C. Passé ce délai, la viscosité de la formulation est mesurée au moyen d'un viscosimètre Brookfield™ à des vitesses de 2,5, 5, 10, 20, 50 et 100 tours par minute, les résultats obtenus étant indiqués dans le tableau 6.

**Tableau 6**

| **Mesure de la viscosité Brookfield™ du lait pour le corps** | |
|---|---|
| **Vitesse de mesure** | **Essai n° 13** (invention) |
| 2,5 tours par minute | 10400 |
| 5 tours par minute | 6700 |
| 10 tours par minute | 4225 |
| 20 tours par minute | 2562 |
| 50 tours par minute | 1285 |
| 100 tours par minute | 752 |

La formulation témoin réalisée selon l'essai n° 14 s'est avérée trop fluide et il a été impossible de déterminer la valeur de la viscosité et ce, quelle que soit la vitesse de mesure.

Les résultats obtenus pour l'essai n° 13 démontrent l'effet épaississant très marqué obtenu par la mise en oeuvre du polymère selon l'invention, à un pH compris entre 6,2 et 6,3, par rapport à la formulation témoin.

### Exemple 4

Cet exemple illustre le procédé selon l'invention, en vue d'épaissir une composition aqueuse qui est une formulation cosmétique de crème de jour anti âge, par introduction dans ladite composition à épaissir d'un polymère contenant un monomère organophosphaté.

Cet exemple illustre également le procédé selon l'invention dans lequel ledit polymère est mis en oeuvre sous forme de dispersion aqueuse.

Cet exemple illustre également le procédé selon l'invention dans lequel le phénomène d'épaississement intervient à un pH acide compris entre 5,5 et 5,6.

Enfin, cet exemple illustre aussi la composition aqueuse selon l'invention qui est une crème de jour, et qui contient ledit polymère.

### Essai n° 15

Pour l'essai n° 15 illustrant l'invention, on réalise une formulation de crème de jour dont la composition est donnée dans le tableau 7.

Cet essai illustre l'invention et met en oeuvre une dispersion aqueuse constituée de :
- 80 % en poids d'eau,
- 20 % en poids d'un polymère de poids moléculaire égal à 129 000 g/mole,
ledit polymère étant constitué de, exprimé en pourcentage en poids des monomères :
35 % d'un mélange de formule (Ia) et (Ib) dans lesquelles R₁ = CH₃, R₂ = H, R₃ = H, n + m = 100,
a) 30,0 % d'acide méthacrylique,
b) 24,8 % d'acrylate d'éthyle,
c) 10,2 % d'un monomère de formule (II) avec :
R désignant le groupement méthacrylate,
R₁ et R₂ désignant l'hydrogène,
n+m+p=25
R' désignant le radical hydrocarboné ayant 22 atomes de carbone.

**Tableau 7**

| **Composition de l'émulsion pour 100 g de produit fini** | | |
|---|---|---|
| **Ordre d'introduction** | **Noms INCI des composés** | **Masse (g)** |
| A | Dimethicone | 4,00 |
| A | Ethylhexylmethoxycinnamate | 5,00 |
| A | Benzophenone-3 | 1,00 |
| A | Ethoxydiglycol Oleate | 6,00 |
| A | Tocopheryl Acetate | 0,50 |
| A | Cetyl Alcohol | 2,50 |
| A | Stearyl Alcohol | 2,50 |
| A | Propylene Glycol Laurate (and) Ethylcellulose (and) Propylene Glycol Isostearate | 6,00 |
| B | Eau déminéralisée | 56,40 |
| B | Dispersion aqueuse contenant le polymère épaississant | 2,45 |
| C | Aluminum Starch Octenylsuccinate | 4,00 |
| C | Glycerin | 2,00 |
| C | Butylene Glycol | 2,00 |
| C | Dimethicone (and) Dimethiconol | 2,00 |
| D | Perfume | 0,15 |
| D | Yellow 6 | 0,40 |
| D | Water (and) Fagus Sylvatica Bud Extract | 3,00 |
| E | Aminomethyl Propanol (qsp pH 5,5 - 5,6) | 0,10 |
| TOTAL | | 100,00 |

Les composés correspondant à l'ordre d'introduction [A] sont pesés puis fondus et homogénéisés à 70°C.

Sous agitation, les composés correspondant à l'ordre d'introduction [B] sont alors ajoutés au mélange qui est ensuite émulsionné au moyen d'une turbine à grande vitesse (6 000 tours par minute).

La température du mélange est alors abaissée à 50°C puis les composés correspondant à l'ordre d'introduction [C] sont alors ajoutés tout en maintenant l'agitation.

Toujours sous agitation, les composés correspondant à l'ordre d'introduction [D] sont alors ajoutés puis le pH est ajusté à une valeur comprise entre 5,5 et 5,6 au moyen du composé correspondant à l'ordre d'introduction [E].

### Essai n° 16

Cet essai correspond à un témoin, pour lequel on a réalisé la même composition de crème de jour que celle indiquée pour l'essai n° 15, à la différence que ladite composition ne contient pas le polymère avec un monomère organophosphaté (on a introduit la même quantité d'eau déminéralisée à la place de la dispersion aqueuse contenant le polymère épaississant).

La formulation finie est stockée dans un flacon fermé pendant 24 heures à 25°C. Passé ce délai, la viscosité de la formulation est mesurée au moyen d'un viscosimètre Brookfield™ à des vitesses de 2.5, 5, 10, 20, 50 et 100 tours par minute, les résultats obtenus étant indiqués dans le tableau 8.

**Tableau 8**

| **Mesure de la viscosité Brookfield™ de la crème de jour** | | |
|---|---|---|
| **Vitesse de mesure** | **Essai n° 16** (témoin) | **Essai n° 15** (invention) |
| 2,5 tours par minute | 23000 | 32000 |
| 5 tours par minute | 17500 | 20000 |
| 10 tours par minute | 9750 | 12750 |
| 20 tours par minute | 5750 | 7500 |
| 50 tours par minute | 3000 | 4200 |
| 100 tours par minute | 1600 | 2600 |

Les résultats du tableau 8 démontrent l'effet épaississant très marqué obtenu à un pH compris entre 5,5 et 5,6 dans le cas de l'invention, c'est-à-dire par la mise en oeuvre du polymère contenant le monomère organophosphaté.

### Exemple 5

Cet exemple illustre le procédé selon l'invention, en vue d'épaissir un gel dans l'eau, par introduction d'un polymère contenant un monomère organophosphaté.

Cet exemple illustre également le procédé selon l'invention dans lequel ledit polymère est mis en oeuvre sous forme de dispersion aqueuse.

Cet exemple illustre notamment la capacité des polymères selon l'invention à développer un effet épaississant à des pH inférieurs aux pH pour lesquels les polymères de l'art antérieur développe cet effet.

Pour chacun des essais n° 17 à 20, on prépare une formulation aqueuse ayant une teneur finale en polymère épaississant de 3 % en poids sec de polymère par rapport au poids total de la formulation, en procédant comme indiqué ci-dessous.

Dans un bécher de 600 ml, on pèse 15 g en poids sec de polymère épaississant à tester et 500 g qsp d'eau désionisée.

Le mélange obtenu est ensuite placé sous agitation modérée afin d'assurer un mélange correct sans toutefois incorporer d'air au milieu.

### Essai n° 17

Cet essai illustre l'art antérieur et met en oeuvre un polymère qui est un copolymère acrylique commercialisé par la société NOVEON™ sous le nom de Carbopol™ Aqua SF1.

### Essai n° 18

Cet essai illustre l'art antérieur et met en oeuvre un polymère qui est un terpolymère de l'acide méthacrylique, de l'acrylate d'éthyle et du diméthacrylate d'éthylène glycol.

### Essai n° 19

Cet essai illustre l'invention et met en oeuvre une dispersion aqueuse constituée de :
- 80 % en poids d'eau,
- 20 % en poids d'un polymère de poids moléculaire égal à 132 000 g/mole,
   ledit polymère étant constitué de, exprimé en pourcentage en poids des monomères :
   35 % d'un mélange de monomères de formule (Ia) et de formule (Ib) dans lesquelles R₁ = CH₃, R₂ = H, n = 1, m = 0
      a) 30,0 % d'acide méthacrylique,
      b) 24,8 % d'acrylate d'éthyle,
      c) 10,2 % d'un monomère de formule (II) avec :
         R désignant le groupement méthacrylate,
         R₁ et R₂ désignant l'hydrogène,
         n + m + p = 25
         R' désignant le radical hydrocarboné ayant 22 atomes de carbone.

### Essai n° 20

Cet essai illustre l'invention et met en oeuvre une dispersion aqueuse constituée de :
- 80 % en poids d'eau,
- 20 % en poids d'un polymère de poids moléculaire égal à 131 500 g/mole,
   ledit polymère étant constitué de, exprimé en pourcentage en poids des monomères :
   35 % d'un mélange de monomères de formule (Ia) et de formule (Ib) dans lesquelles R₁ = CH₃, R₂ = H, n = 1, m = 0
      a) 28,9 % d'acide méthacrylique,
      b) 23,6 % d'acrylate d'éthyle,
      c) 12,5 % d'un monomère de formule (II) avec :
         R désignant le groupement méthacrylate,
         R₁ et R₂ désignant l'hydrogène,
         n + m + p = 25
         R' désignant le radical hydrocarboné ayant 22 atomes de carbone.

Le pH de la formulation est contrôlé en continu au moyen d'un pH mètre.

On ajoute alors de petites fractions d'hydroxyde de sodium (10 %), on mélange jusqu'à stabilisation du pH et on mesure la viscosité Brookfield™ à 100 tours par minute (voir tableau 9).

Cette opération est répétée jusqu'à ce que la viscosité augmente rapidement, la courbe adoptant une trajectoire quasi verticale. La montée de viscosité permet clairement de déterminer le pH à partir duquel le polymère viscosifie le milieu.

Les mesures de viscosité sont reportées dans le tableau 9.

**Tableau 9**

| **Essai n° 17** Art antérieur | | **Essai n° 18** Art antérieur | | **Essai n° 19** Polymère de l'invention | | **Essai n° 20** Polymère de l'invention | |
|---|---|---|---|---|---|---|---|
| pH | Viscosité Brookfield™ 100 tours par minute (mPa.s) | pH | Viscosité Brookfield™ 100 tours par minute (mPa.s) | pH | Viscosité Brookfield™ 100 tours par minute (mPa.s) | pH | Viscosité Brookfield™ 100 tours par minute (mPa.s) |
| 5,00 | 20,00 | 5,54 | 12,00 | 5,00 | 30,00 | 5,00 | 32,00 |
| 6,10 | 20,00 | 5,95 | 26,00 | 5,20 | 40,00 | 5,20 | 40,00 |
| 6,25 | 64,00 | 6,12 | 70,00 | 5,35 | 74,00 | 5,30 | 50,00 |
| 6,35 | 176,00 | 6,15 | 220,00 | 5,50 | 480,00 | 5,40 | 80,00 |
| 6,40 | 725,00 | 6,18 | 690,00 | 5,60 | 610,00 | 5,45 | 260,00 |
| - | - | 6,20 | 1100,00 | 5,65 | 1360,00 | 5,56 | 1800,00 |

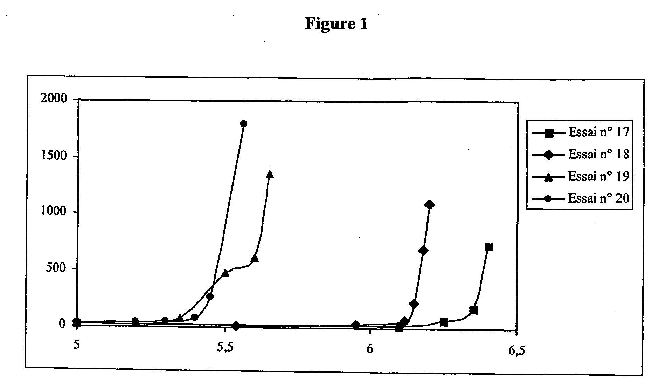

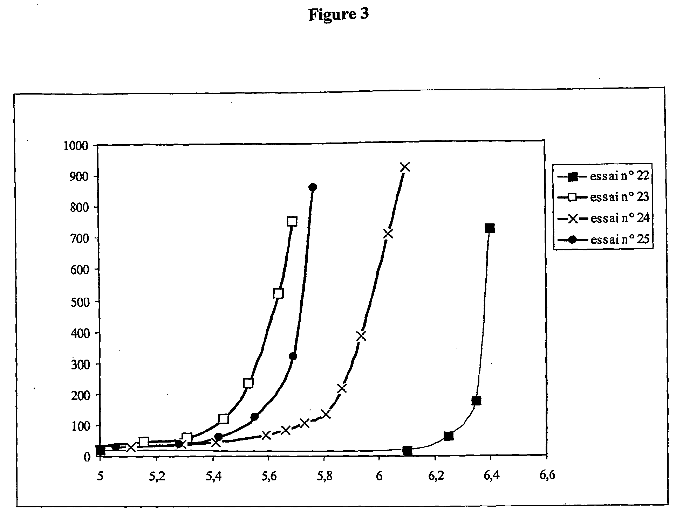

Le tableau 9 permet de représenter l'évolution de la viscosité en fonction du pH, pour chacun des essais n° 17 à 20 : la figure 1 / 3 représente l'évolution de la viscosité Brookfield™ mesurée à 100 tours par minute en fonction du pH.

On voit ainsi clairement que l'effet épaississant intervient pour les polymères selon l'invention, à des pH bien inférieurs à ceux pour lesquels intervient l'effet épaississant des polymères selon l'art antérieur.

### Exemple 6

Cet exemple illustre le procédé selon l'invention, en vue d'épaissir un gel dans l'eau, par introduction d'un polymère contenant un monomère organophosphaté.

Cet exemple illustre également le procédé selon l'invention dans lequel ledit polymère est mis en oeuvre sous forme de dispersion aqueuse.

Cet exemple illustre notamment la capacité des polymères selon l'invention à développer un effet épaississant à des pH inférieurs aux pH pour lesquels les polymères de l'art antérieur développe cet effet.

Cet exemple illustre également le procédé selon l'invention dans la variante selon laquelle on met en oeuvre la technique de retour-acide.

La procédure suivante permet de quantifier la capacité d'un polymère de l'invention à développer son pouvoir épaississant à un niveau de pH satisfaisant en utilisant la technique du rétour acide. Selon cette méthode, le pH de la formulation contenant le polymère est amené à une valeur d'environ 8 puis redescendu jusqu'à un pH de 4. Cette technique permet de mettre en évidence le pH utile du polymère.

### Essai n° 21

Pour ce faire, on prépare une formulation aqueuse en procédant comme suit :
Dans un bécher de 600 ml, on pèse 25 g d'une dispersion aqueuse du polymère à tester et 500 qsp d'eau désionisée, le polymère à tester étant un polymère selon l'invention.
Ladite dispersion aqueuse est constituée de :
   - 80 % en poids d'eau,
   - 20 % en poids d'un polymère de poids moléculaire égal à 128 000 g/mole,
      ledit polymère étant constitué de, exprimé en pourcentage en poids des monomères :
      35 % d'un mélange de monomères de formule (Ia) et de formule (Ib) dans lesquelles R₁ = CH₃, R₂ = H, n = 1, m = 0
         a) 30,0 % d'acide méthacrylique,
         b) 24,8 % d'acrylate d'éthyle,
         c) 10,2 % d'un monomère de formule (II) avec :
            R désignant le groupement méthacrylate,
            R₁ et R₂ désignant l'hydrogène,
            n + m + p = 25
            R' désignant le radical hydrocarboné ayant 22 atomes de carbone.

Le mélange obtenu est ensuite placé sous agitation modérée afin d'assurer un mélange correct sans toutefois incorporer d'air au milieu.

Le pH de la formulation est contrôlé en continu au moyen d'un pH mètre.

On ajoute alors de petites fractions d'hydroxyde de sodium (10 %), on mélange jusqu'à stabilisation du pH et on mesure la viscosité Brookfield™ de la formulation à 100 tours par minute. Cette opération est répétée jusqu'à ce que le pH atteigne une valeur d'environ 8. Une fois cette valeur atteinte, le pH est redescendu jusqu'à une valeur d'environ 4 au moyen d'acide lactique, ajouté par petites fractions, le pH et la viscosité Brookfield™ à 100 tours par minute étant mesurés après chaque ajout d'acide.

**Tableau 10**

| **pH de la solution** | **Viscosité Brookfield™ 100 tours par minute (mPa.s)** |
|---|---|
| 4,78 | 17 |
| 5,54 | 26 |
| 5,85 | 39 |
| 6 | 61 |
| 6,15 | 106 |
| 6,26 | 200 |
| 6,45 | 380 |
| 6,62 | 458 |
| 7,1 | 550 |
| 8,3 | 624 |
| 8,3 | 624 |
| 7,53 | 500 |
| 6,71 | 240 |
| 6,55 | 187 |
| 6,32 | 145 |
| 6,01 | 117 |
| 5,5 | 68 |
| 5,5 | 68 |
| 4,98 | 50 |

Les résultats sont reportés dans le tableau 10.

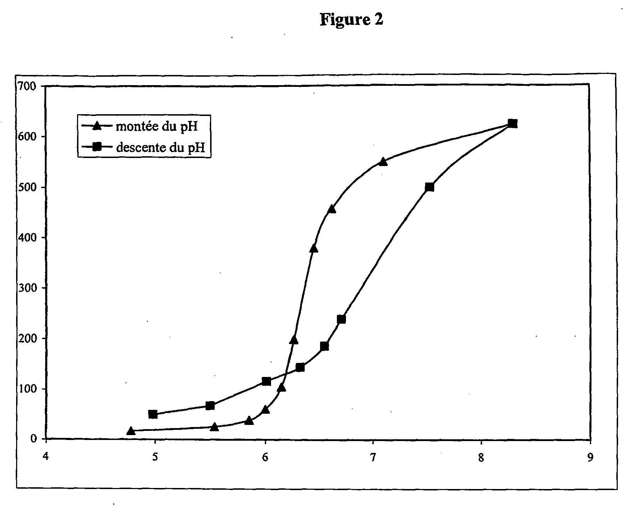

Ils permettent d'établir la figure 2 / 3 qui représente l'évolution en fonction du pH, de la viscosité Brookfield mesurée à 100 tours par minute, pendant la période où le pH augmente, et pendant la période où le pH diminue.

Cette figure illustre l'effet épaississant développé par le polymère selon l'invention, lorsqu'il est mise en oeuvre dans le cadre de la technique dite de retour-acide.

### Exemple 7

Cet exemple illustre le procédé selon l'invention, en vue d'épaissir un gel dans l'eau, par introduction d'un polymère contenant un monomère organophosphaté.

Cet exemple illustre également le procédé selon l'invention dans lequel ledit polymère est mis en oeuvre sous forme de dispersion aqueuse.

Cet exemple illustre notamment la capacité des polymères selon l'invention à développer un effet épaississant à des pH inférieurs aux pH pour lesquels les polymères de l'art antérieur développent cet effet.

Pour chacun des essais n° 22 à 25, on prépare une formulation aqueuse ayant une teneur finale en polymère épaississant de 3 % en poids sec de polymère par rapport au poids total de la formulation, en procédant comme indiqué ci-dessous.

Dans un bécher de 600 ml, on pèse 15 g en poids sec de polymère épaississant à tester et 500 g qsp d'eau désionisée.

Le mélange obtenu est ensuite placé sous agitation modérée afin d'assurer un mélange correct sans toutefois incorporer d'air au milieu.

### Essai n° 22

Cet essai illustre l'art antérieur et met en oeuvre un polymère qui est un copolymère acrylique commercialisé par la société NOVEON™ sous le nom de Carbopol™ Aqua SF1.

### Essai n° 23

Cet essai illustre l'invention et met en oeuvre une dispersion aqueuse constituée de :
- 80 % en poids d'eau,
- 20 % en poids d'un polymère de poids moléculaire égal 141 000 à g/mole,
   ledit polymère étant constitué de, exprimé en pourcentage en poids des monomères :
   35 % d'un mélange de monomères de formule (Ia) et de formule (Ib) dans lesquelles R₁ = CH₃, R₂ = H, n = 1, m = 0
      a) 32,4 % d'acide méthacrylique et 3,6 % d'acide acrylique,
      b) 23,0 % d'acrylate d'éthyle,
      c) 6,0 % d'un monomère de formule (II) avec :
         R désignant le groupement méthacrylate,
         R₁ et R₂ désignant l'hydrogène,
         n + m + p = 25
         R' désignant le radical hydrocarboné ayant 22 atomes de carbone.

### Essai n° 24

Cet essai illustre l'invention et met en oeuvre une dispersion aqueuse constituée de :
- 80 % en poids d'eau,
- 20 % en poids d'un polymère de poids moléculaire égal à 140 500 g/mole,
   ledit polymère étant constitué de, exprimé en pourcentage en poids des monomères :
   35 % d'un mélange de monomères de formule (Ia) et de formule (Ib) dans lesquelles R₁ = CH₃, R₂ = H, n = 1, m = 0
      a) 36,0 % d'acide méthacrylique,
      b) 23,0 % d'acrylate d'éthyle,
      c) 6,0 % d'un monomère de formule (II) avec :
         R désignant le groupement méthacrylate,
         R₁ et R₂ désignant l'hydrogène,
         n + m + p = 25
         R' désignant un radical hydrocarboné ramifié ayant 16 atomes de carbone.

### Essai n° 25

Cet essai illustre l'invention et met en oeuvre une dispersion aqueuse constituée de :
- 80 % en poids d'eau,
- 20 % en poids d'un polymère de poids moléculaire égal à 137 500 g/mole,
   ledit polymère étant constitué de, exprimé en pourcentage en poids des monomères :
   35 % d'un mélange de monomères de formule (Ia) et de formule (Ib) dans lesquelles R₁ = CH₃, R₂ = H, n = 1, m = 0
      a) 34,0 % d'acide méthacrylique et 2,0 % de diméthacrylate d'éthylène glycol,
      b) 23,0 % d'acrylate d'éthyle,
      c) 6,0 % d'un monomère de formule (II) avec :
         R désignant le groupement méthacrylate,
         R₁ et R₂ désignant l'hydrogène,
         n + m + p = 25
         R' désignant le radical hydrocarboné ayant 22 atomes de carbone.

La figure 3 / 3 représente l'évolution de la viscosité Brookfield™ mesurée à 100 tours par minute en fonction du pH.

On voit ainsi clairement que l'effet épaississant intervient pour les polymères selon l'invention, à des pH bien inférieurs à ceux pour lesquels intervient l'effet épaississant des polymères selon l'art antérieur.

## Revendications

1. - Procédé d'épaississement d'une composition aqueuse, par introduction dans ladite composition à épaissir d'au moins un polymère, **caractérisé en ce que** ledit polymère possède un poids moléculaire superieur à 80 000 g/mole et contient au moins un monomère anionique qui est un monomère organophosphaté.

2. - Procédé selon la revendication 1, **caractérisé en ce que** le monomère organophosphaté est choisi parmi les molécules de formules : 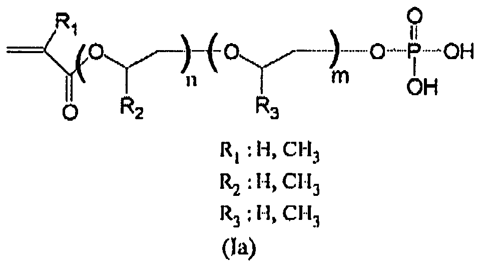 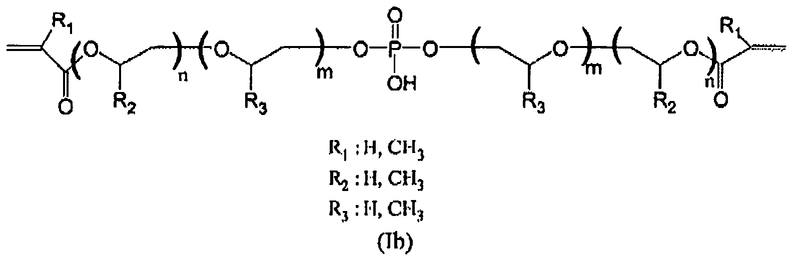 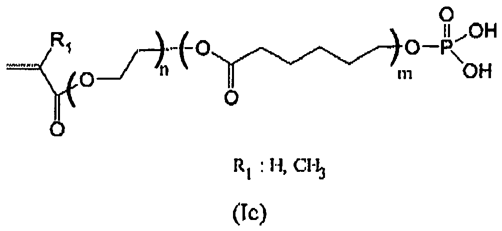 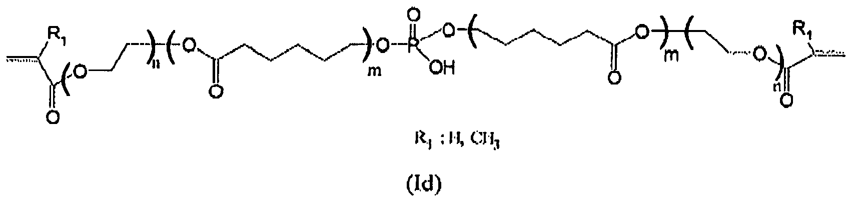 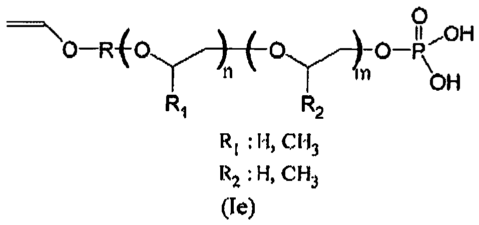 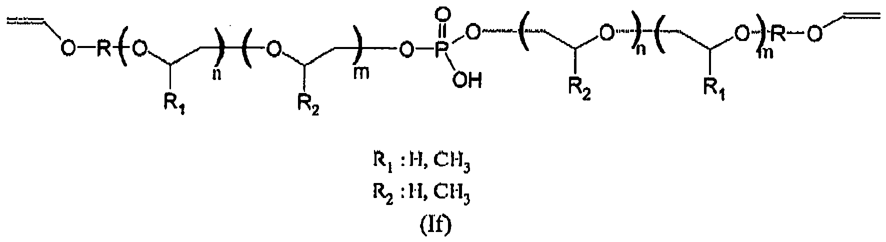 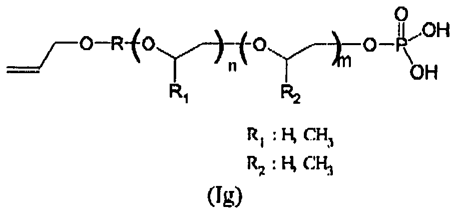 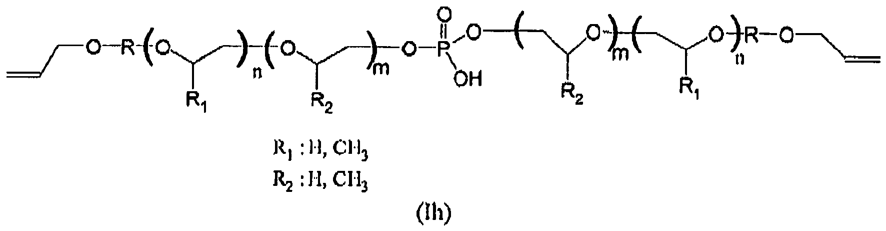 et leurs mélanges, avec n désignant un entier compris entre 1 et 100, préférentiellement entre 1 et 20, m désignant un entier compris entre 0 et 100, préférentiellement entre 0 et 20, et R désignant une chaîne alkyle ayant de 2 à 8 atomes de carbone.

3. - Procédé selon l'une des revendications 1 ou 2, **caractérisé en ce que** la composition à épaissir possède un pH compris entre 5 et 7, préférentiellement entre 5 et 6,5, très préférentiellement entre 5,5 et 6.

4. - Procédé selon l'une des revendications 1 à 3, **caractérisé en ce que** ledit polymère est introduit dans la composition aqueuse à épaissir sous forme de poudre, et / ou sous forme de dispersion aqueuse, et / ou sous forme de dispersion solvantée, et / ou sous forme de dispersion inverse, et / ou sous forme de solution aqueuse, et / ou sous forme de solution solvantée.

5. - Procédé selon l'une des revendications 1 à 4, **caractérisé en ce qu'**il met en oeuvre la technique retour-acide, c'est-à-dire qu'il comprend les étapes d'introduction dudit polymère dans la composition aqueuse à épaissir, d'introduction d'un composé alcalin permettant d'augmenter la valeur de pH à une valeur supérieure à 5, préférentiellement 6, très préférentiellement 6,5, puis de diminution de la valeur du pH par un composé acide à une valeur inférieure à 7, préférentiellement à 6,5, très préférentiellement inférieure à 5,5.

6. - Procédé selon l'une des revendications 1 à 5, **caractérisé en ce que** ledit polymère contient éventuellement:
a) au moins un autre monomère anionique différent du monomère organophosphaté,
b) et / ou au moins un monomére non-ionique vinylique,
c) et / ou au moins un monomère non-ionique à groupement hydrophobe,
d) et / ou au moins un monomère organofluoré ou organosililé ou leurs mélanges,
e) et / ou au moins un monomère réticulant, c'est-à-dire un monomère ayant au moins 2 liaisons polymérisables, ledit monomère étant différent des monomères organophosphatés de formules (Ib), (Id), (If), et (Ih).

7. - Procédé selon la revendication 6, **caractérisé en ce que** ledit polymère contient, exprimé en pourcentage en poids de chacun des constituants, de 0,01 à 100 %, préférentiellement de 10 à 100 %, très préférentiellement de 20 à 100 % du monomère organophosphaté, et :
a) de 0 à 90 % d'au moins un autre monomère anionique différent du monomère organophosphaté,
b) de 0 à 50 % d'au moins un monomère non-ionique vinylique,
c) de 0 à 20 % d'au moins un monomère non-ionique tel groupement hydrophobe,
d) de 0 à 10 % d'au moins un monomère organofluoré ou organosililé ou leurs mélanges,
e) de 0 à 5 % d'au moins un monomère réticulant, c'est-à-dire un monomère ayant au moins 2 liaisons polymérisables, ledit monomère étant différent des monomères organophosphatés de formules (Ib), (Id), (If), et (Ih).
la somme des pourcentages en poids des monomères constituant ledit polymère étant égale à 100.

8. - Procédé selon l'une des revendications 6 ou 7, **caractérisé en ce que** le monomère anionique a) est un monomère à insaturation éthylénique et à fonction carboxylique choisi parmi les monomères à insaturation éthylénique et à fonction monocarboxylique et est alors préférentiellement l'acide acrylique, méthacrylique, crotonique, isocrotonique, cinnamique ou leurs mélanges, ou choisi parmi les hémiesters de diacides et est alors préférentiellement un monoester en C₁ à C₄ des acides maléique ou itaconique, ou leurs mélanges, ou choisi parmi les monomères à insaturation éthylénique et fonction dicarboxylique à l'état acide ou salifié, et préférentiellement parmi l'acide, itaconique, maléique, fumarique, mésaconique ou leurs mélanges ou encore choisi parmi les anhydrides d'acides carboxyliques, et est alors préférentiellement l'anhydride maléique.

9. - Procédé selon l'une des revendications 6 ou 7, **caractérisé en ce que** le monomère non-ionique vinylique b) est choisi parmi les esters, les amides ou les nitriles des acides acrylique et méthacrylique, et est alors très préférentiellement choisi parmi les acrylates
ou méthacrylates de méthyle, éthyle, butyle, 2-éthyle-hexyle, et leurs mélanges ou est choisi parmi l'acrylonitrile, l'acétate de vinyle, le styrène, le méthylstyrène, le diisobutylène, la vinylpyrrolidone, la vinylcaprolactame et leurs mélanges.

10. - Procédé selon l'une des revendications 6 ou 7, **caractérisé en ce que** le monomère non-ionique à groupement hydrophobe c), est un monomère de formule (II) : 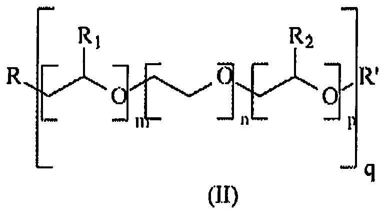 dans laquelle :
- m et p représentent un nombre de motifs d'oxyde d'alkylène inférieur ou égal à 150,
- n représente un nombre de motifs d'oxyde d'éthylène inférieur ou égal à 150,
- q représente un nombre entier au moins égal à 1 et tel que 5 ≤ (m+n+p)q ≤ 150, et préférentiellement tel que 15 ≤ (m+n+p)q ≤ 120,
- R₁ représente l'hydrogène ou le radical méthyle ou éthyle,
- R₂ représente l'hydrogène ou le radical méthyle ou éthyle,
- R représente un radical contenant une fonction insaturée polymérisable, appartenant préférentiellement au groupe des vinyliques ainsi qu'au groupe des esters acrylique, méthacrylique, maléique, itaconique, crotonique, vinylphtalique ainsi qu'au groupe des insaturés uréthannes tels que les acryluréthanne, méthacryluréthanne, α-α' dimethyl-isopropenylbenzyluréthanne, allyluréthanne, de même qu'au groupe des éthers allyliques ou vinyliques substitués ou non, ou encore au groupe des amides ou des imides éthyléniquement insaturées,
- R' représente l'hydrogène ou un radical hydrocarboné ayant 5 à 50 atomes de carbone, et représente préférentiellement un radical hydrocarboné ayant 12 à 50 atomes de carbone et très préférentiellement un radical hydrocarboné ayant 16 à 36 atomes de carbone,

11. - Procédé selon l'une des revendications 6 ou 7, **caractérisé en ce que** le monomère réticulant e) est choisi dans le groupe constitué par le diméthacrylate d'éthylène glycol, le triméthylolpropanetriacrylate, l'acrylate d'allyle, les maléates d'allyle, le méthyléne-bis-acrylamide, le méthylène-bis-méthacrylamide, le tétrallyloxyéthane, les triallylcyanurates, les éthers allyliques obtenus à partir de polyols tels que le pentaérythritol, le sorbitol, le sucrose, ou choisi parmi les molécules de formule (IV) : 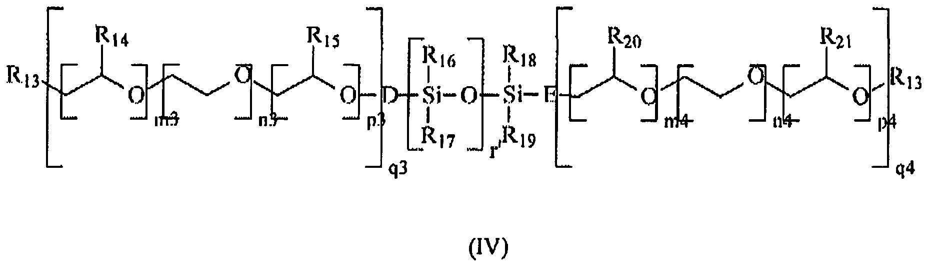 dans laquelle :
- m₃, p₃, m₄ et p₄ représentent un nombre de motifs d'oxyde d'alkylène inférieur ou égal à 150,
- n₃ et n₄ représentent un nombre de motifs d'oxyde d'éthylène inférieur ou égal à 150,
- q₃ et q₄ représentent un nombre entier au moins égal à 1 et tel que 0 ≤ (m₃-n₃-p₃)q₃ ≤ 150 et 0 ≤ (m₄+n₄+p₄)q₄ ≤ 150,
- r' représente un nombre tel que 1 ≤ r' ≤ 200,
- R₁₃ représente un radical contenant une fonction insaturée polymérisable, appartenant préférentiellement au groupe des vinyliques ainsi qu'au groupe des esters acrylique, méthacrylique, maléique, itaconique, crotonique, vinylphtalique ainsi qu'au groupe des insaturés uréthannes tels que les acryluréthanne, méthacryluréthanne, α-α' diméthyl-isopropénylbenzyluréthanne, allyluréthanne, de même qu'au groupe des éthers allyliques ou vinyliques substitués ou non, ou encore au groupe des amides ou des imides éthyténiquement insaturées,
- R₁₄, R₁₅, R₂₀ et R₂₁, représentent l'hydrogène ou le radical méthyle ou méthyle,
- R₁₆, R₁₇, R₁₈ et R₁₉, représentent des groupements linéaires ou ramifiés alkyle, ou aryle, ou alkylaryle, ou arylalkyle ayant 1 à 20 atomes de carbone, ou leur mélange,
- D et E sont des groupements éventuellement présents, qui représentent
alors un radical hydrocarboné ayant 1 à 4 atomes de carbone,
ou parmi les mélanges de ces molécules.

12. - Procédé selon l'une des revendications 1 à 11, **caractérisé en ce que** ledit polymère possède un poids moléculaire supérieur à 100 000 g/mole et préférentiellement supérieur à 120 000 g/mole.

13. - Compositions aqueuses épaissies, **caractérisées en ce qu'**elles contiennent comme épaississante un polymère contenant au moins un monomère anionique qui est un monomère organophosphaté, ledit polymère ayant un poids moléculaire supérieur à 80 000 g/mole, préférentiellement supérieur à 100 000 g/mole, très préférentiellement supérieur à 120 000 g/mole

14. - Compositions aqueuses selon la revendication 13, **caractérisées en ce que** le monomère organophosphaté est choisi parmi les molécules de formules : 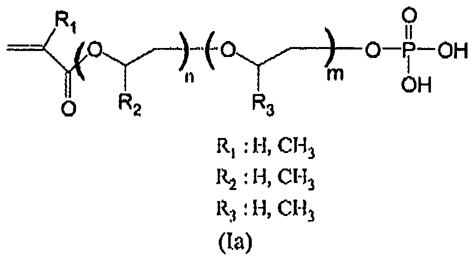 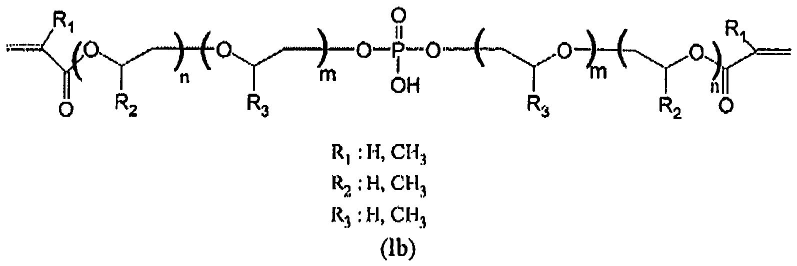 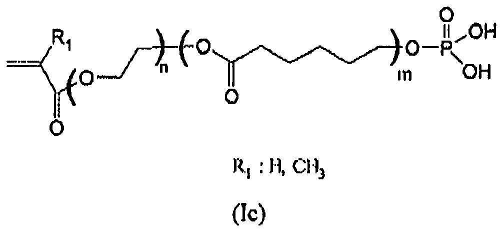 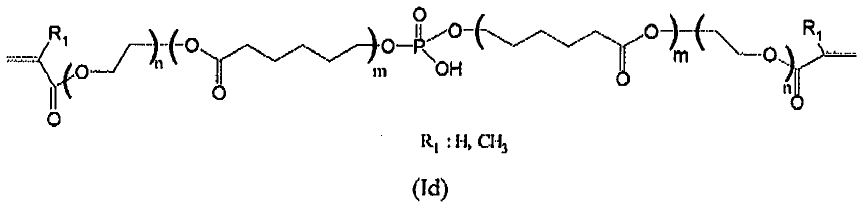 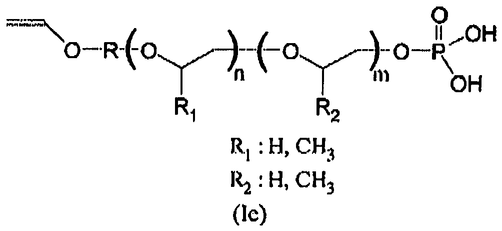 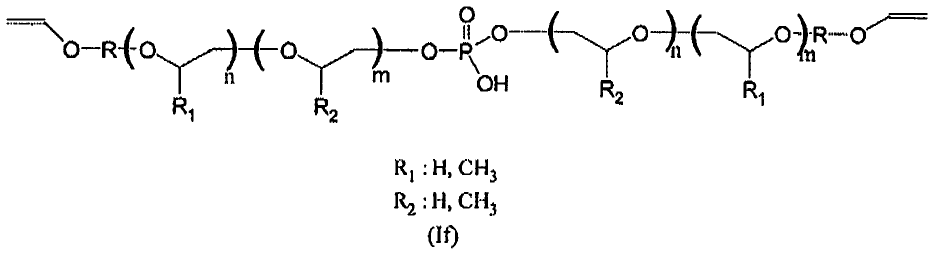 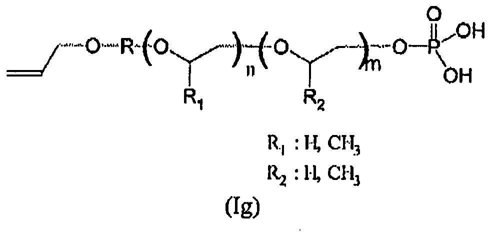 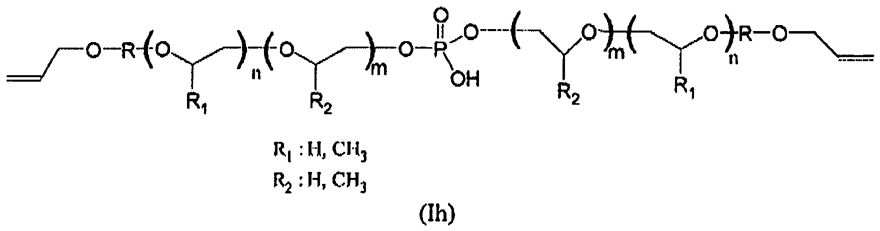 et leurs mélanges, avec n désignant un entier compris entre 1 et 100, préférentiellement entre 1 et 20, m désignant un entier compris entre 0 et 100, préférentiellement entre 0 et 20, et R désignant une chaîne alkyle ayant de 2 à 8 atomes de carbone.

15. - Compositions aqueuses selon l'une des revendications 13 ou 14, **caractérisées en ce qu'**elles possèdent un pH compris entre 5 et 7, préférentiellement entre 5 et 6,5, très préférentiellement entre 5,5 et 6.

16. - Compositions aqueuses selon l'une des revendications 13 à 15, **caractérisées en ce que** ledit polymère épaississant qu'elles contiennent, contient éventuellement :
a) au moins un autre monomère anionique différent du monomère organophosphaté,
b) et / ou au moins un monomère non-ionique vinylique,
c) et / ou au moins un monomère non-ionique à groupement hydrophobe,
d) et / ou au moins un monomère organofluoré ou organosililé ou leurs mélanges,
e) et / ou au moins un monomère réticulant, c'est-à-dire un monomère ayant au moins 2 liaisons polymérisable, ledit monomère étant différent des monomères organophosphatés de formules (Ib), (Id), (If), et (Ih).

17. - Compositions aqueuses selon la revendication 16, **caractérisées en ce que** ledit polymère épaississant qu'elles contiennent contient, exprimé en pourcentage en poids de chacun des constituants, de 0,01 à 100 %, préférentiellement de 10 à 100 %, très préférentiellement de 20 à 100 % du monomère organophosphaté, et :
a) de 0 à 90 % d'au moins un autre monomère anionique différent du monomère organophosphaté,
b) de 0 à 50 % d'au moins un monomère non-ionique vinylique,
c) de 0 à 20 % d'au moins un monomère non-ionique à groupement hydrophobc,
d) de 0 à 10 % d'au moins un monomère organofluoré ou organosililé ou leurs mélanges,
e) de 0 à 5 % d'au moins un monomère réticulant, c'est-à-dire un monomère ayant au moins 2 liaisons polymérisabics, ledit monomère étant différent des monomères organophosphatés de formules (Ib), (Id), (If), et (Ih).
la somme des pourcentages en poids des monomères constituant ledit polymère étant égale à 100.

18. - Compositions aqueuses, selon l'une des revendications 16 ou 17, **caractérisées en ce que** le mononmère anionique a) du polymère épaississant qu'elles contiennent, est un monomère à insaturation éthylénique et à fonction carboxylique choisi parmi les monomères à insaturation éthylénique et à fonction monocarboxylique et est alors préférentiellement l'acide acrylique, méthacrylique, crotonique, isocrotonique, cinnamique ou leurs mélanges, ou choisi parmi les hémiesters de diacides et est alors préférentiellement un monoester en C₁ à C₄ des acides maléique ou itaconique, ou leurs mélanges, ou choisi parmi les monomères à insaturation éthylénique et fonction dicarboxylique à l'état acide ou salifié, et préférentiellement parmi l'acide, itaconique, maléique, fumarique, mésaconique ou leurs mélanges ou encore choisi parmi les anhydrides d'acides carboxyliques, et est alors préférentiellement l'anhydride maléique.

19. - Compositions aqueuses, selon l'une des revendications 16 ou 17, **caractérisées en ce que** le monomère non-ionique vinylique b) du polymère épaississant qu'elles contiennent, est choisi parmi les esters, les amides ou les nitrites des acides acrylique et méthacrylique, et est alors très préférentiellement choisi parmi les acrylates ou méthacrylates de méthyle, éthyle, butyle, 2-éthyle-hexyle, et leurs mélanges ou est choisi parmi l'acrylonitrile, l'acétate de vinyle, le styrène, le méthylstyrène, le diisobutylène, la vinylpyrrolidone, la vinylcaprolactame et leurs mélanges.

20. - Compositions aqueuses, selon l'une des revendications 16 ou 17, **caractérisées en ce que** le monomère non-ionique à groupement hydrophobe c) du polymère épaississant qu'elles contiennent, est un monomère de formule (II) : 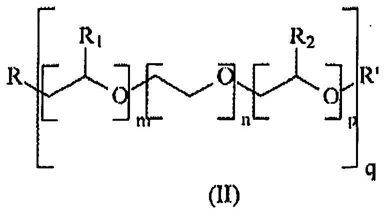 dans laquelle:
- m et p représentent un nombre de motifs d'oxyde d'alkylène inférieur ou égal à 150,
- n représente un nombre de motifs d'oxyde d'éthylène inférieur ou égal à 150,
- q représente un nombre entier au moins égal à 1 et tel que 5 ≤ (m+n+p)q ≤ 150, et préférentiellement tel que 15 ≤ (m+n+p)q ≤ 120,
- R₁ représente l'hydrogène ou le radical méthyle ou éthyle,
- R₂ représente l'hydrogène ou le radical méthyle ou éthyle,
- R représente un radical contenant une fonction insaturée polymérisable, appartenant préférentiellement au groupe des vinyliques ainsi qu'au groupe des esters acrylique, méthacrylique, maléique, itaconique, crotonique, vinylphtalique ainsi qu'au groupe des insaturés uréthannes tels que les acryluréthanne, méthacryluréthanne, α-α' diméthyl-isopropénylbenzyluréthanne, allyluréthanne, de même qu'au groupe des éthers allyliques ou vinyliques substitués ou non, ou encore au groupe des amides ou des imides éthyléniquement insaturées,
- R' représente l'hydrogène ou un radical hydrocarboné ayant 5 à 50 atomes de carbone, et représente préférentiellement un radical hydrocarboné ayant 12 à 50 atomes de carbone et très préférentiellement un radical hydrocarboné ayant 16 à 36 atomes de carbone.

21. - Compositions aqueuses, selon l'une des revendications 16 ou 17, **caractérisées en ce que** le monomère réticulant c) du polymère épaississant qu'elles contiennent, est choisi dans le groupe constitué par le diméthacrylate d'éthylène glycol, le triméthylolpropanetriacrylate, l'acrylate d'allyle, les maléates d'allyle, le méthyléne-bis-acrylamide, le méthylène-bis-méthacrylamide, le tétrallyloxyéthane, les triallylcyanurates, les éthers allyliques obtenus à partir de polyols tels que le pentaérythritol, le sorbitol, le sucrose, ou choisi parmi les molécules de formule (IV) : 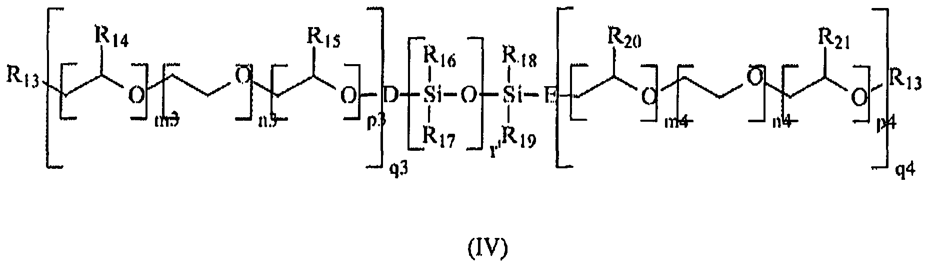 dans laquelle :
- m₃, p₃, m₄ et p₄ représentent un nombre de motifs d'oxyde d'alkylène inférieur ou égal à 150,
- n₃ et n₄ représentent un nombre de motifs d'oxyde d'éthylène inférieur ou égal à 150,
- q₃ et q₄ représentent un nombre entier au moins égal à 1 et tel que 0 ≤ (m₃+n₃+p₃)q₃ ≤ 150 et 0 ≤ (m₄+n₄+p₄)q₄ ≤ 150,
- r' représente un nombre tel que 1 ≤ r' ≤ 200,
- R₁₃ représente un radical contenant une fonction insaturée polymérisable, appartenant préférentiellement au groupe des vinyliques ainsi qu'au groupe des esters acrylique, méthacrylique, maléique, itaconique, crotonique, vinylphtalique ainsi qu'au groupe des insaturés uréthannes tels que les acryluréthanne, methacryluréthanne, α-α' diméthyl-isopropénylbenzyluréthanne, allyluréthanne, de même qu'au groupe des éthers allyliques ou vinyliques substitués ou non, ou encore au groupe des amides ou des imides éthyléniquement insaturées,
- R₁₄, R₁₅, R₂₀ et R₂₁, représentent l'hydrogène ou le radical méthyle ou éthyle,
- R₁₆, R₁₇, R₁₈ et R₁₉, représentent des groupements linéaires ou ramifiés alkyle, ou aryle, ou alkylaryle, ou arylalkyle ayant 1 à 20 atomes de carbone, ou leur mélange,
- D et E sont des groupements éventuellement présents, qui représentent
alors un radical hydrocarboné ayant 1 à 4 atomes de carbone,
ou parmi les mélanges de ces molécules.

22. - Compositions aqueuses, selon l'une des revendications 16 à 21, **caractérisées en ce qu'**elles sont des compositions cosmétiques, pharmaceutiques, des compositions à base de liants hydrauliques et sont alors préférentiellement des bétons, des ciments, des mortiers, des coulis, des laitiers, des compositions détergentes, des sauces de couchage papetières, des peintures.

## Patentansprüche

1. - Verfahren zur Eindickung einer wässrigen Zusammensetzung, indem der einzudickenden Zusammensetzung mindestens ein Polymer zugesetzt wird, **dadurch gekennzeichnet, dass** das Polymer ein Molekulargewicht von mehr als 80.000 g/mol besitzt und mindestens ein anionisches Monomer enthält, bei welchem es sich um ein organophosphatartiges Monomer handelt.

2. - Verfahren nach Anspruch 1, **dadurch gekennzeichnet, dass** das organophosphatartige Monomer aus den Molekülen nach den folgenden Formeln sowie deren Mischungen ausgewählt ist: 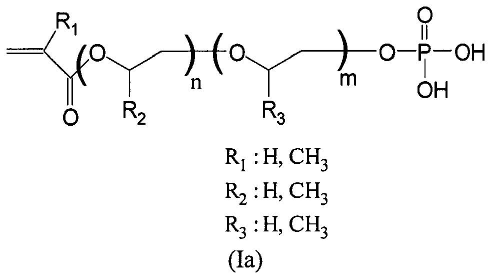 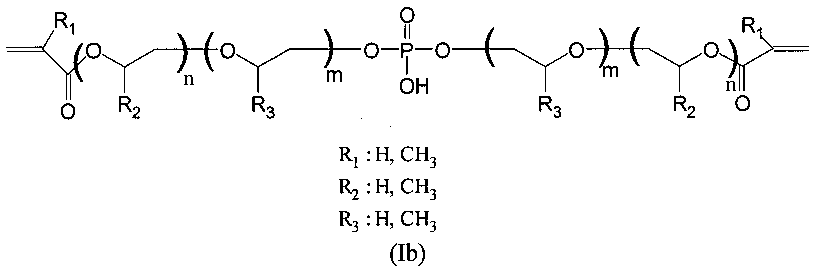 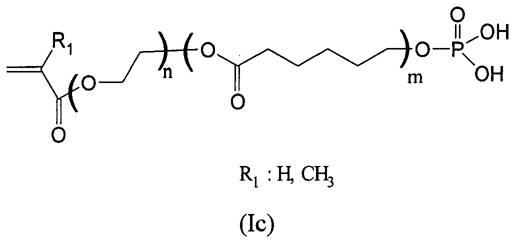 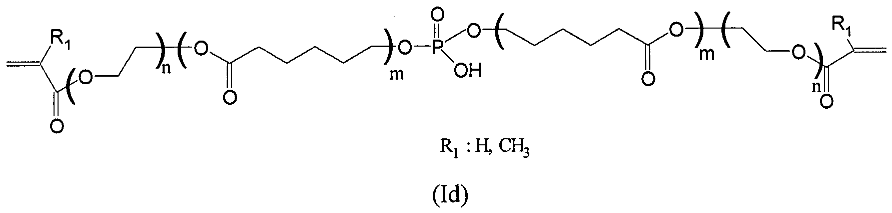 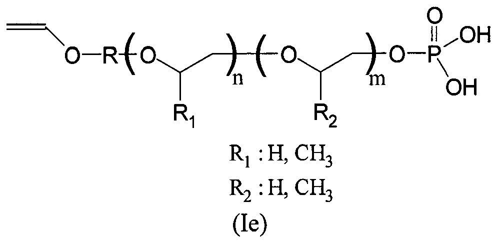 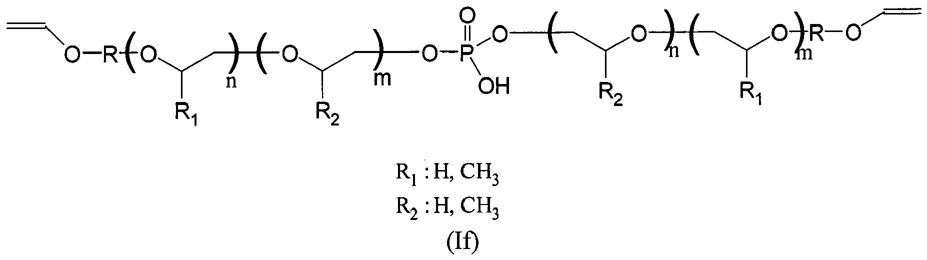 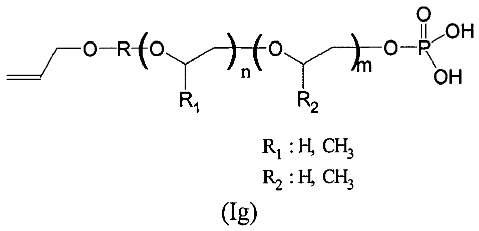 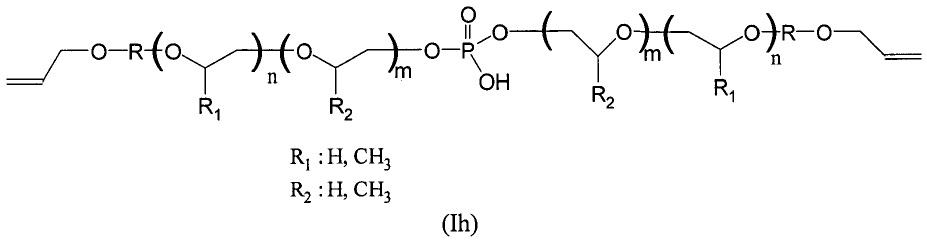 wobei n für eine ganze Zahl im Bereich von 1 bis 100, vorzugsweise von 1 bis 20, steht, m für eine ganze Zahl im Bereich von 0 bis 100, vorzugsweise von 0 bis 20, steht und R für eine Alkylkette mit 2 bis 8 Kohlenstoffatomen steht.

3. - Verfahren nach einem der Ansprüche 1 oder 2, **dadurch gekennzeichnet, dass** die einzudickende Zusammensetzung einen pH-Wert im Bereich von 5 bis 7, vorzugsweise von 5 bis 6,5, mit starkem Vorzug von 5,5 bis 6, besitzt.

4. - Verfahren nach einem der Ansprüche 1 bis 3, **dadurch gekennzeichnet, dass** das Polymer der einzudickenden wässrigen Zusammensetzung in Form eines Pulvers und/oder in Form einer wässrigen Dispersion und/oder in Form einer lösungsmittelbasierten Dispersion und/oder in Form einer Umkehrdispersion und/oder in Form einer wässrigen Lösung und/oder in Form einer lösungsmittelbasierten Lösung zugesetzt wird.

5. - Verfahren nach einem der Ansprüche 1 bis 4, **dadurch gekennzeichnet, dass** die Technik der Wiederansäuerung zum Einsatz kommt, das heißt, dass es die Schritte des Zusatzes des Polymers zu der einzudickenden wässrigen Zusammensetzung, des Zusatzes einer alkalischen Verbindung, die es ermöglicht, den pH-Wert auf einen Wert von mehr als 5, vorzugsweise 6, mit starkem Vorzug 6,5 zu erhöhen, wobei der pH-Wert anschließend mittels einer sauren Verbindung auf einen Wert von weniger als 7, vorzugsweise als 6,5, mit starkem Vorzug als 5,5, abgesenkt wird.

6. - Verfahren nach einem der Ansprüche 1 bis 5, **dadurch gekennzeichnet, dass** das Polymer möglicherweise Folgendes enthält:
a) mindestens ein weiteres anionisches Monomer, das sich vom organophosphatartigen Monomer unterscheidet,
b) und/oder mindestens ein vinylartiges nichtionisches Monomer,
c) und/oder mindestens ein nichtionisches Monomer mit einer hydrophoben Gruppe,
d) und/oder mindestens ein organofluorartiges oder organosilylartiges Monomer oder deren Mischungen,
e) und/oder mindestens ein vernetzendes Monomer, das heißt, ein Monomer mit mindestens 2 polymerisierbaren Bindungen, wobei dieses Monomer sich von den organophosphatartigen Monomeren nach den Formeln (Ib), (Id), (If) und (Ih) unterscheidet.

7. - Verfahren nach Anspruch 6, **dadurch gekennzeichnet, dass** das Polymer, ausgedrückt in Gewichtsprozentanteilen jedes seiner Bestandteile, 0,01 bis 100 %, vorzugsweise 10 bis 100 %, mit starkem Vorzug 20 bis 100 %, des organophosphatartigen Monomers enthält, und
a) 0 bis 90 % eines weiteren anionischen Monomers, das sich vom organophosphatartigen Monomer unterscheidet,
b) 0 bis 50 % mindestens eines vinylartigen nichtionischen Monomers,
c) 0 bis 20 % mindestens eines nichtionischen Monomers mit hydrophober Gruppe,
d) 0 bis 10 % mindestens eines organofluorartigen oder organosilylartigen Monomers oder von deren Mischungen,
e) 0 bis 5 % mindestens eines vernetzenden Monomers, das heißt, eines Monomers mit mindestens 2 polymerisierbaren Bindungen, wobei dieses Monomer sich von den organophosphatartigen Monomeren nach den Formeln (Ib), (Id), (If) und (Ih) unterscheidet.
wobei die Summe der Gewichtsprozentanteile der Monomere, aus denen das Polymer besteht, gleich 100 ist.

8. - Verfahren nach einem der Ansprüche 6 bis 7, **dadurch gekennzeichnet, dass** es sich bei dem anionischen Monomer a) um ein ethylenisch ungesättigtes Monomer mit einer Carbonsäurefunktion handelt, wobei dieses aus den ethylenisch ungesättigten Monomeren mit Monocarbonsäurefunktion und zwar vorzugweise aus Acrylsäure, Methacrylsäure, Crotonsäure, Isocrotonsäure, Zimtsäure oder deren Mischungen ausgewählt ist, oder aus den Halbestern von Disäuren und zwar vorzugsweise aus einem C₁- bis C₄-Monoester von Maleinsäure oder Itaconsäure oder deren Mischungen ausgewählt ist, oder aus den ethylenisch ungesättigten Monomeren mit Dicarbonsäurefunktion in Säureform oder in versalzter Form und zwar vorzugsweise aus Itaconsäure, Maleinsäure, Fumarsäure, Mesaconsäure oder deren Mischungen ausgewählt ist, oder aber aus den Carbonsäureanhydriden und zwar vorzugsweise aus Maleinsäureanhydrid ausgewählt ist.

9. - Verfahren nach einem der Ansprüche 6 oder 7, **dadurch gekennzeichnet, dass** das vinylartige nichtionische Monomer b) aus den Estern, den Amiden oder den Nitrilen von Acrylsäure und Methacrylsäure und zwar mit starkem Vorzug aus den Methyl-, Ethyl-, Butyl-, 2-Ethylhexylacrylaten oder -methacrylaten und deren Mischungen ausgewählt ist oder aus Acrylnitril, Vinylacetat, Styrol, Methylstyrol, Diisobutylen, Vinylpyrrolidon, Vinylcaprolactam und deren Mischungen ausgewählt ist.

10. - Verwendung nach einem der Ansprüche 6 bis 7, **dadurch gekennzeichnet, dass** es sich bei dem nichtionischen Monomer mit hydrophober Gruppe c) um ein Monomer nach der folgenden Formel (II) handelt: 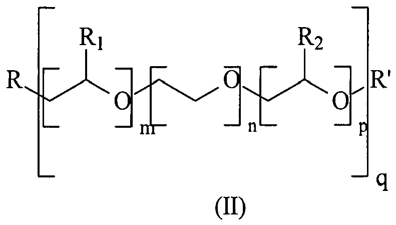 wobei:
- m und p für eine Anzahl an Alkylenoxidbausteinen von höchstens 150 stehen,
- n für eine Anzahl an Ethylenoxidbausteinen von höchstens 150 steht,
- q für eine ganze Zahl steht, die mindestens gleich 1 und derart ist, dass 5 ≤ (m+n+p)q ≤ 150, und vorzugsweise derart ist, dass 15 ≤ (m+n+p)q ≤ 120,
- R₁ für Wasserstoff oder einen Methyl- oder Ethylrest steht,
- R₂ für Wasserstoff oder einen Methyl- oder Ethylrest steht,
- R für einen polymerisierbaren ungesättigten Rest steht, welcher vorzugsweise der Gruppe der Vinylverbindungen oder auch der Gruppe der Acrylsäure-, Methacrylsäure-, Maleinsäure-, Itaconsäure-, Crotonsäure-, Vinylphthalsäureester oder auch der Gruppe der ungesättigten Urethanverbindungen, wie etwa Acrylurethan, Methacrylurethan, α-α'-Dimethylisopropenylbenzylurethan, Allylurethan, wie auch der Gruppe der substituierten oder unsubstituierten Allyl- oder Vinylether oder aber der Gruppe der ethylenisch ungesättigten Amide oder Imide angehört,
- R' für Wasserstoff oder einen Kohlenwasserstoffrest mit 5 bis 50 Kohlenstoffatomen steht, und vorzugsweise für einen Kohlenwasserstoffrest mit 12 bis 50 Kohlenstoffatomen sowie mit starkem Vorzug für einen Kohlenwasserstoffrest mit 16 bis 36 Kohlenstoffatomen steht,

11. - Verfahren nach einem der Ansprüche 6 oder 7, **dadurch gekennzeichnet, dass** das vernetzende Monomer e) aus der Gruppe ausgewählt ist, die aus Ethylenglycoldimethacrylat, Trimethylolpropantriacrylat, Allylacrylat, den Allylmaleaten, Methylen-bis-acrylamid, Methylen-bis-methacrylamid, Tetrallyloxyethan, den Triallylcyanuraten, den Allylethern, welche ausgehend von Polyolen wie etwa Pentaerythrit, Sorbit, Saccharose erhalten werden, besteht, oder aus den Molekülen nach der Formel (IV) ausgewählt ist: 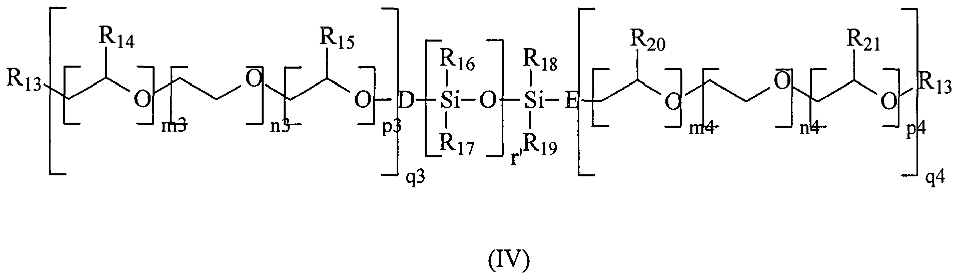 wobei:
- m₃, p₃, m₄ und p₄ für eine Anzahl an Alkylenoxidbausteinen von höchstens 150 stehen,
- n₃ und n₄ für eine Anzahl an Ethylenoxidbausteinen von höchstens 150 stehen,
- q₃ und q₄ für eine ganze Zahl stehen, die mindestens gleich 1 ist und derart ist, dass 0 ≤ (m₃+n₃+p₃)q₃ ≤ 150 und 0 ≤ (m₄+n₄+p₄)q₄ ≤ 150,
- r' für eine Anzahl steht, die derart ist, dass 1 ≤ r' ≤ 200,
- R₁₃ für einen Rest steht, der eine polymerisierbare ungesättigte funktionelle Gruppe enthält, wobei er vorzugsweise der Gruppe der Vinylverbindungen oder auch der Gruppe der Acrylsäure-, Methacrylsäure-, Maleinsäure-, Itaconsäure-, Crotonsäure-, Vinylphthalsäureester oder auch der Gruppe der ungesättigten Urethanverbindungen, wie etwa Acrylurethan, Methacrylurethan, α-α'-Dimethylisopropenylbenzylurethan, Allylurethan, wie auch der Gruppe der substituierten oder unsubstituierten Allyl- oder Vinylether oder aber der Gruppe der ethylenisch ungesättigten Amide oder Imide angehört,
- R₁₄, R₁₅, R₂₀ und R₂₁ für Wasserstoff oder einen Methyl- oder Ethylrest stehen,
- R₁₆, R₁₇, R₁₈ und R₁₉ für geradkettige oder verzweigte Alkyl- oder Aryl- oder Alkylaryl- oder Arylalkylgruppen mit 1 bis 20 Kohlenstoffatomen oder deren Mischung stehen,
- D und E Gruppen darstellen, die vorhanden sein können und in diesen Falle
für einen Kohlenwasserstoffrest mit 1 bis 4 Kohlenstoffatomen stehen,
oder aus den Mischungen dieser Moleküle.

12. - Verfahren nach einem der Ansprüche 1 bis 11, **dadurch gekennzeichnet, dass** das Polymer ein Molekulargewicht von mehr als 100.000 g/mol und vorzugsweise von mehr als 120.000 g/mol besitzt.

13. - Eingedickte wässrige Zusammensetzungen, **dadurch gekennzeichnet, dass** sie als Verdickungsmittel ein Polymer enthalten, das mindestens ein anionisches Monomer enthält, bei welchem es sich um ein organophosphatartiges Monomer handelt, wobei das Polymer ein Molekulargewicht von mehr als 80.000 g/mol, vorzugsweise von 100.000 g/mol, mit starkem Vorzug von mehr als 120.000 g/mol hat.

14. - Wässrige Zusammensetzungen nach Anspruch 13, **dadurch gekennzeichnet, dass** das organophosphatartige Monomer aus den Molekülen nach den folgenden Formeln oder deren Mischungen ausgewählt ist: 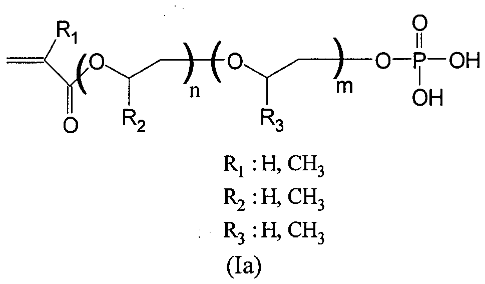 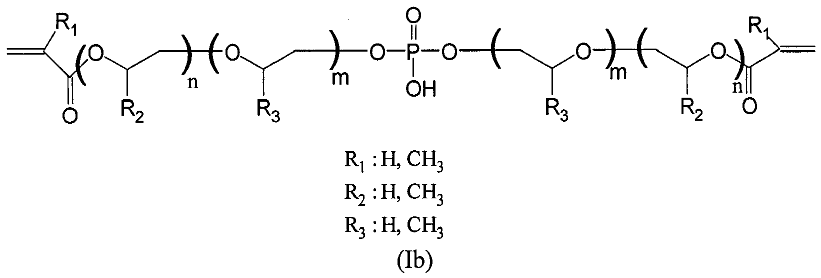 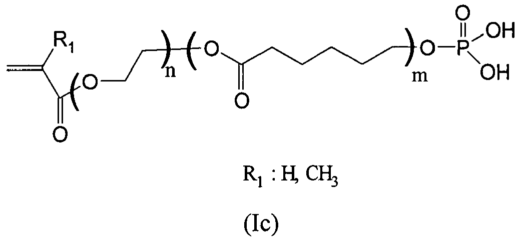 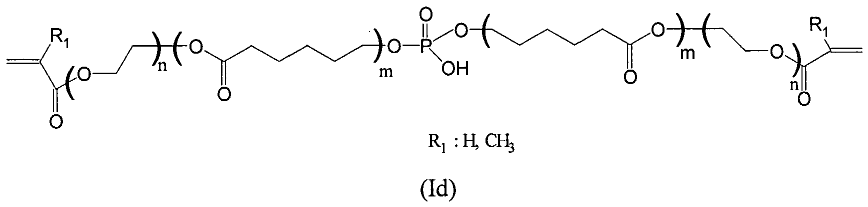 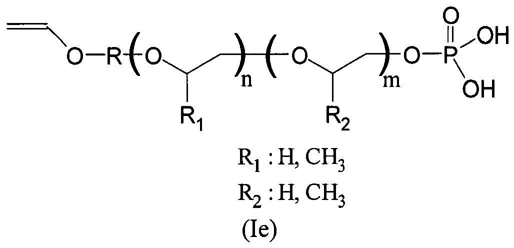 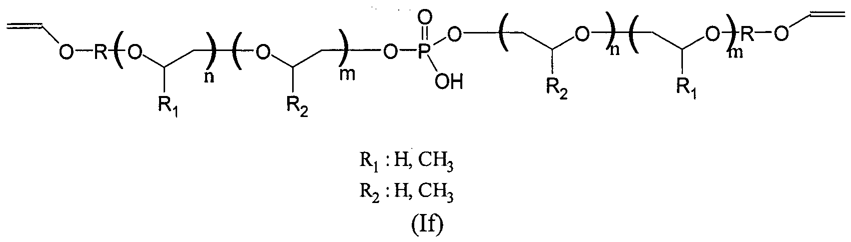 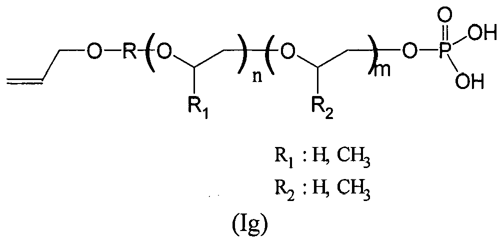 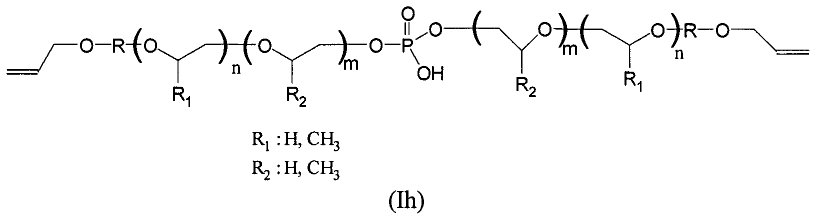 wobei n für eine ganze Zahl im Bereich von 1 bis 100, vorzugsweise von 1 bis 20, steht, m für eine ganze Zahl im Bereich von 0 bis 100, vorzugsweise von 0 bis 20, steht und R für eine Alkylkette mit 2 bis 8 Kohlenstoffatomen steht.

15. - Wässrige Zusammensetzungen nach einem der Ansprüche 13 oder 14, **dadurch gekennzeichnet, dass** sie einen pH-Wert im Bereich von 5 bis 7, vorzugsweise von 5 bis 6,5, mit starkem Vorzug von 5,5 bis 6, besitzen.

16. - Wässrige Zusammensetzungen nach einem der Ansprüche 13 bis 15, **dadurch gekennzeichnet, dass** das verdickende Polymer, welches sie enthalten, möglicherweise Folgendes enthält:
a) mindestens ein weiteres anionisches Monomer, das sich vom organophosphatartigen Monomer unterscheidet,
b) und/oder mindestens ein vinylartiges nichtionisches Monomer,
c) und/oder mindestens ein nichtionisches Monomer mit einer hydrophoben Gruppe,
d) und/oder mindestens ein organofluorartiges oder organosilylartiges Monomer oder deren Mischungen,
e) und/oder mindestens ein vernetzendes Monomer, das heißt, ein Monomer mit mindestens 2 polymerisierbaren Bindungen, wobei dieses Monomer sich von den organophosphatartigen Monomeren nach den Formeln (Ib), (Id), (If) und (Ih) unterscheidet.

17. - Wässrige Zusammensetzungen nach Anspruch 16, **dadurch gekennzeichnet, dass** das Polymer, welches sie enthalten, ausgedrückt in Gewichtsprozentanteilen jedes seiner Bestandteile, 0,01 bis 100 %, vorzugsweise 10 bis 100 %, mit starkem Vorzug 20 bis 100 % des organophosphatartigen Monomers enthält, und
a) 0 bis 90 % eines weiteren anionischen Monomers, das sich vom organophosphatartigen Monomer unterscheidet,
b) 0 bis 50 % mindestens eines vinylartigen nichtionischen Monomers,
c) 0 bis 20 % mindestens eines nichtionischen Monomers mit hydrophober Gruppe,
d) 0 bis 10 % mindestens eines organofluorartigen oder organosilylartigen Monomer oder von deren Mischungen,
e) 0 bis 5 % mindestens eines vernetzenden Monomers, das heißt, eines Monomers mit mindestens 2 polymerisierbaren Bindungen, wobei dieses Monomer sich von den organophosphatartigen Monomeren nach den Formeln (Ib), (Id), (If) und (Ih) unterscheidet.
wobei die Summe der Gewichtsprozentanteile der Monomere, aus denen das Polymer besteht, gleich 100 ist.

18. - Wässrige Zusammensetzungen nach einem der Ansprüche 16 bis 17, **dadurch gekennzeichnet, dass** es sich bei dem anionischen Monomer a) des verdickenden Polymers, welches sie enthalten, um ein ethylenisch ungesättigtes Monomer mit einer Carbonsäurefunktion handelt, wobei dieses aus den ethylenisch ungesättigten Monomeren mit Monocarbonsäurefunktion und zwar vorzugweise aus Acrylsäure, Methacrylsäure, Crotonsäure, Isocrotonsäure, Zimtsäure oder deren Mischungen ausgewählt ist, oder aus den Halbestern von Disäuren und zwar vorzugsweise aus einem C₁- bis C₄-Monoester von Maleinsäure oder Itaconsäure oder deren Mischungen ausgewählt ist, oder aus den ethylenisch ungesättigten Monomeren mit Dicarbonsäurefunktion in Säureform oder in versalzter Form und zwar vorzugsweise aus Itaconsäure, Maleinsäure, Fumarsäure, Mesaconsäure oder deren Mischungen ausgewählt ist, oder aber aus den Carbonsäureanhydriden und zwar vorzugsweise aus Maleinsäureanhydrid ausgewählt ist.

19. - Wässrige Zusammensetzungen nach einem der Ansprüche 16 oder 17, **dadurch gekennzeichnet, dass** das vinylartige nichtionische Monomer b) des verdickenden Polymers, welches sie enthalten, aus den Estern, den Amiden oder den Nitrilen von Acrylsäure und Methacrylsäure und zwar mit starkem Vorzug aus den Methyl-, Ethyl-, Butyl-, 2-Ethylhexylacrylaten oder -methacrylaten und deren Mischungen ausgewählt ist oder aus Acrylnitril, Vinylacetat, Styrol, Methylstyrol, Diisobutylen, Vinylpyrrolidon, Vinylcaprolactam und deren Mischungen ausgewählt ist.

20. - Wässrige Zusammensetzungen nach einem der Ansprüche 16 bis 17, **dadurch gekennzeichnet, dass** es sich bei dem nichtionischen Monomer mit hydrophober Gruppe c) des verdickenden Polymers, welches sie enthalten, um ein Monomer nach der folgenden Formel (II) handelt: 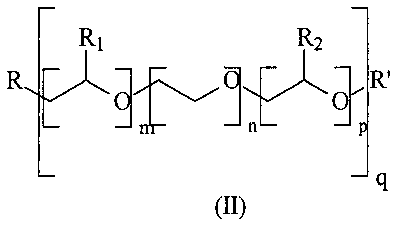 wobei:
- m und p für eine Anzahl an Alkylenoxidbausteinen von höchstens 150 stehen,
- n für eine Anzahl an Ethylenoxidbausteinen von höchstens 150 steht,
- q für eine ganze Zahl steht, die mindestens gleich 1 und derart ist, dass 5 ≤ (m+n+p)q ≤ 150, und vorzugsweise derart ist, dass 15 ≤ (m+n+p)q ≤ 120,
- R₁ für Wasserstoff oder einen Methyl- oder Ethylrest steht,
- R₂ für Wasserstoff oder einen Methyl- oder Ethylrest steht,
- R für einen polymerisierbaren ungesättigten Rest steht, welcher vorzugsweise der Gruppe der Vinylverbindungen oder auch der Gruppe der Acrylsäure-, Methacrylsäure-, Maleinsäure-, Itaconsäure-, Crotonsäure-, Vinylphthalsäureester oder auch der Gruppe der ungesättigten Urethanverbindungen, wie etwa Acrylurethan, Methacrylurethan, α-α'-Dimethylisopropenylbenzylurethan, Allylurethan, wie auch der Gruppe der substituierten oder unsubstituierten Allyl- oder Vinylether oder aber der Gruppe der ethylenisch ungesättigten Amide oder Imide angehört,
- R' für Wasserstoff oder einen Kohlenwasserstoffrest mit 5 bis 50 Kohlenstoffatomen steht, und vorzugsweise für einen Kohlenwasserstoffrest mit 12 bis 50 Kohlenstoffatomen sowie mit starkem Vorzug für einen Kohlenwasserstoffrest mit 16 bis 36 Kohlenstoffatomen steht,

21. - Wässrige Zusammensetzungen nach einem der Ansprüche 16 oder 17, **dadurch gekennzeichnet, dass** das vernetzende Monomer e) des verdickenden Polymers, welches sie enthalten, aus der Gruppe ausgewählt ist, die aus Ethylenglycoldimethacrylat, Trimethylolpropantriacrylat, Allylacrylat, den Allylmaleaten, Methylen-bis-acrylamid, Methylen-bis-methacrylamid, Tetrallyloxyethan, den Triallylcyanuraten, den Allylethern, welche ausgehend von Polyolen wie etwa Pentaerythrit, Sorbit, Saccharose erhalten werden, besteht, oder aus den Molekülen nach der Formel (IV) ausgewählt ist: 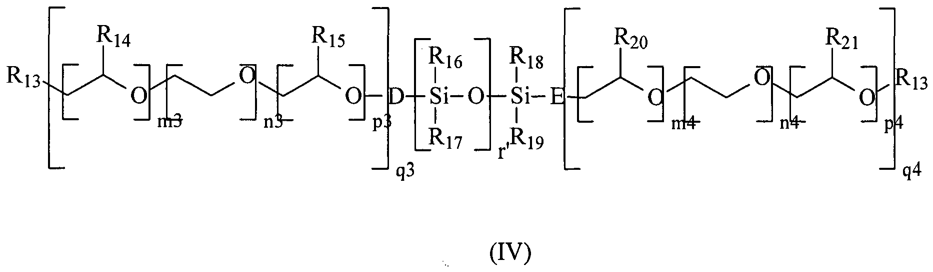 wobei:
- m₃, p₃, m₄ und p₄ für eine Anzahl an Alkylenoxidbausteinen von höchstens 150 stehen,
- n₃ und n₄ für eine Anzahl an Ethylenoxidbausteinen von höchstens 150 stehen,
- q₃ und q₄ für eine ganze Zahl stehen, die mindestens gleich 1 ist und derart ist, dass 0 ≤ (m₃+n₃+p₃)q₃ ≤ 150 und 0 ≤ (m₄+n₄+p₄)q₄ ≤ 150,
- r' für eine Anzahl steht, die derart ist, dass 1 ≤ r' ≤ 200,
- R₁₃ für einen Rest steht, der eine polymerisierbare ungesättigte funktionelle Gruppe enthält, wobei er vorzugsweise der Gruppe der Vinylverbindungen oder auch der Gruppe der Acrylsäure-, Methacrylsäure-, Maleinsäure-, Itaconsäure-, Crotonsäure-, Vinylphthalsäureester oder auch der Gruppe der ungesättigten Urethanverbindungen, wie etwa Acrylurethan, Methacrylurethan, α-α'-Dimethylisopropenylbenzylurethan, Allylurethan, wie auch der Gruppe der substituierten oder unsubstituierten Allyl- oder Vinylether oder aber der Gruppe der ethylenisch ungesättigten Amide oder Imide angehört,
- R₁₄, R₁₅, R₂₀ und R₂₁ für Wasserstoff oder einen Methyl- oder Ethylrest stehen,
- R₁₆, R₁₇, R₁₈ und R₁₉ für geradkettige oder verzweigte Alkyl- oder Aryl- oder Alkylaryl- oder Arylalkylgruppen mit 1 bis 20 Kohlenstoffatomen oder deren Mischung stehen,
- D und E Gruppen darstellen, die vorhanden sein können und in diesen Falle
für einen Kohlenwasserstoffrest mit 1 bis 4 Kohlenstoffatomen stehen,
oder aus den Mischungen dieser Moleküle.

22. - Wässrige Zusammensetzungen nach einem der Ansprüche 16 bis 21, **dadurch gekennzeichnet, dass** es sich dabei um kosmetische, pharmazeutische Zusammensetzungen, um Zusammensetzungen auf Grundlage hydraulischer Bindemittel und zwar vorzugsweise um Betonsorten, Zementsorten, Mörtelsorten, Schlämme, Schlacken, um reinigend wirkende Zusammensetzungen, um Papierstreichmassen, um Anstrichmittel handelt.

## Claims

1. - Process for thickening an aqueous composition, by introducing into said composition to be thickened at least one polymer, **characterised in that** said polymer has a molecular weight greater than 80,000 g/mol and contains at least one anionic monomer, which is an organophosphate monomer.

2. - The process according to claim 1, **characterised in that** the organophosphate monomer is chosen among molecules with the following formulas: 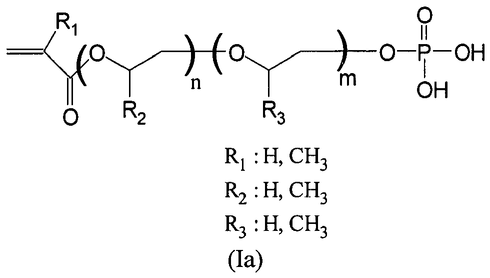 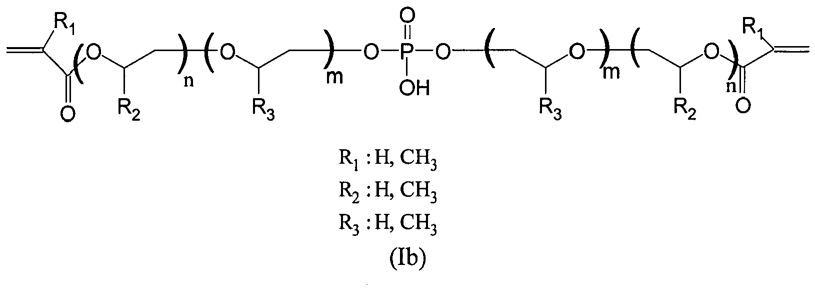 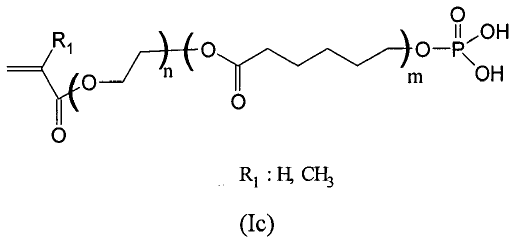 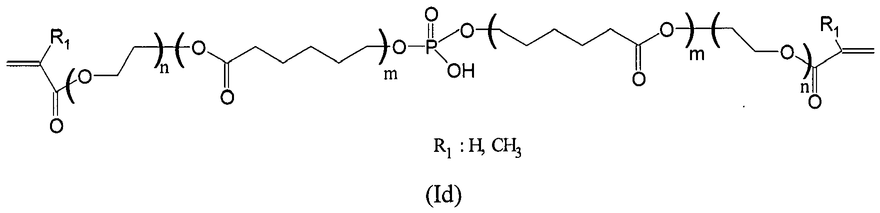 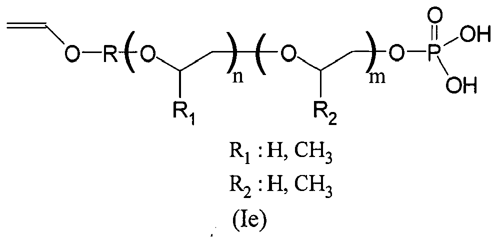 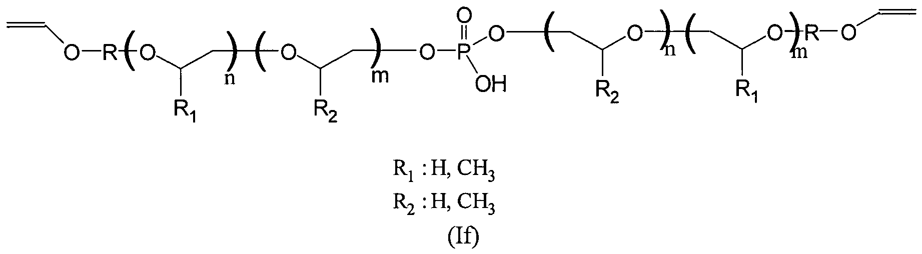 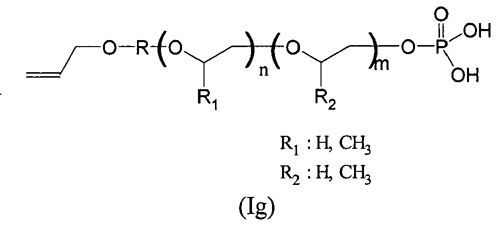 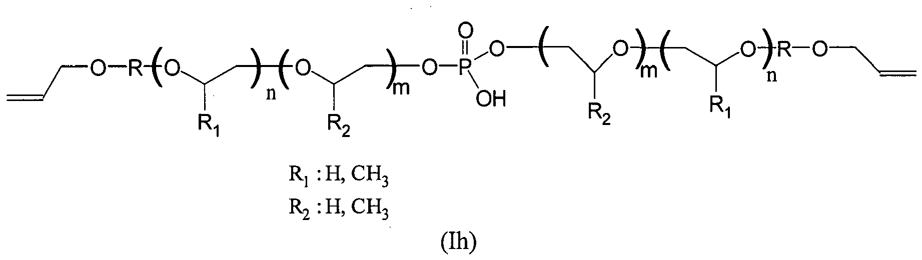 and their blends, with n designating an integer between 1 and 100, preferentially between 1 and 20, with m designating an integer between 0 and 100, preferentially between 0 and 20, and R designating an alkyl chain of 2 to 8 carbon atoms.

3. - The process according to one of claims 1 or 2, **characterised in that** said composition to be thickened has a pH between 5 and 7, preferentially between 5 and 6.5, very preferentially between 5.5 and 6.

4. - The process according to one of claims 1 to 3, **characterised in that** said polymer is introduced into the aqueous composition to be thickened in powder form, and/or in the form of an aqueous dispersion, and/or in the form of a solvent-based dispersion, and/or in the form of a reverse dispersion, and/or in the form of an aqueous solution, and/or in the form of a solvent-based solution.

5. - The process according to one of claims 1 to 4, **characterised in that** it uses the back-acid technique, i.e. it includes the stages of introduction of said polymer into the aqueous composition to be thickened, of introduction of an alkaline compound which increases the pH value to a value greater than 5, preferentially 6, and very preferentially 6.5, and of reduction of the pH value by means of an acid compound to a value of less than 7, preferentially 6.5, and very preferentially less than 5.5.

6. - The process according to one of claims 1 to 5, **characterised in that** said polymer possibly contain:
a) at least one other anionic monomer different from the organophosphate monomer,
b) and/or at least one vinylic non-ionic monomer,
c) and/or at least one non-ionic monomer with hydrophobic grouping,
d) and/or at least one organofluorate or organosililate monomer or their blends,
e) and/or at least one cross-linking monomer, i.e. a monomer having at least 2 polymerisable links, where said monomer is different from the organophosphate monomers of formulae (Ib), (Id), (If), and (Ih).

7. - The process according to claim 6, **characterised in that** said polymer contains, expressed as a percentage by weight of each of the constituents, from 0.01 to 100%, preferentially from 10 to 100%, and very preferentially from 20 to 100%, of the organophosphate monomer, and:
a) from 0 to 90% of at least one other anionic monomer different from the organophosphate monomer,
b) from 0 to 50% of at least one non-ionic vinylic monomer,
c) from 0 to 20% of at least one non-ionic monomer with hydrophobic group,
d) from 0 to 10% of at least one organofluorate or organosililate monomer or their blends,
e) from 0 to 5% of at least one cross-linking monomer, i.e. a monomer having at least 2 polymerisable links, where said monomer is different from the organophosphate monomers of formulae (Ib), (Id), (If), and (Ih).
the sum of the percentages by weight of monomers constituting said polymer is equal to 100.

8. - The process according to one of claims 6 or 7, **characterised in that** the anionic monomer a) is a monomer with ethylenic unsaturation and with a carboxylic group, chosen among the monomers with ethylenic unsaturation and with a monocarboxylic group, and is then preferentially acrylic, methacrylic, crotonic, isocrotonic or cinnamic acid, or their blends, or chosen among the hemiesters of diacids, and is then preferentially a monoester at C₁ to C₄ of maleic or itaconic acids, or their blends, or chosen among the monomers with ethylenic unsaturation and with a dicarboxylic group in the acid or salified state, and preferentially among itaconic, maleic, fumaric or mesaconic acid, or their blends, or chosen among the anhydrides of carboxylic acids, and is then preferentially maleic anhydride.

9. - The process according to one of claims 6 or 7, **characterised in that** the vinylic non-ionic monomer b) is chosen among esters, amides or nitriles of acrylic and methacrylic acids, and is then very preferentially chosen among the methyl, ethyl, butyl or 2-ethyl-hexyl acrylates or methacrylates, and their blends, or is chosen among acrylonitrile, vinyl acetate, styrene, methylstyrene, di-isobutylene, vinylpyrrolidone, vinylcaprolactame and their blends.

10. - The process according to one of claims 6 or 7, **characterised in that** the non-ionic monomer with hydrophobic grouping c) is a monomer of formula (II): 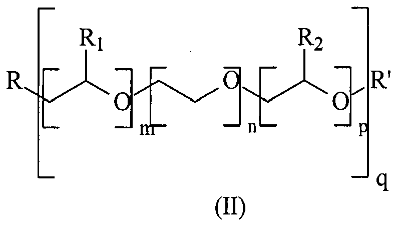 where:
- m and p represent a number of alkylene oxide units less than or equal to 150,
- n represents a number of ethylene oxide units less than or equal to 150,
- q represents an integer of at least 1 and such that 5 ≤ (m+n+p)q ≤ 150, and preferentially such that 15 ≤ (m+n+p)q ≤ 120,
- R₁ represents hydrogen or a methyl or ethyl radical,
- R₂ represents hydrogen or a methyl or ethyl radical,
- R represents a radical containing an unsaturated polymerisable function, preferentially belonging to the group of vinylics, or to the group of acrylic, methacrylic, maleic, itaconic, crotonic or vinylphthalic esters, or to the group of unsaturated urethanes such as acrylurethane, methacrylurethane, α-α' dimethyl-isopropenyl-benzylurethane, allylurethane, or to the group of allylic or vinylic ethers, whether substituted or not, or to the group of ethylenically unsaturated amides or imides,
- R' represents hydrogen or a hydrocarbonate radical having 5 to 50 atoms of carbon, and represents preferentially a hydrocarbonate radical having 12 to 50 carbon atoms, and very preferentially a hydrocarbonate radical with 16 to 36 carbon atoms,

11. -The process according to one the claims 6 or 7, **characterised in that** the cross-linking monomer e) is selected in the group consisting of ethylene glycol dimethacrylate, trimethylolpropanetriacrylate, allyl acrylate, the allyl maleates, methylene-bis-acrylamide, methylene-bis-methacrylamide, tetrallyloxyethane, the triallylcyanurates, the allyl ethers obtained from polyols such as pentaerythritol, sorbitol, sucrose, or chosen among the molecules of formula (IV): 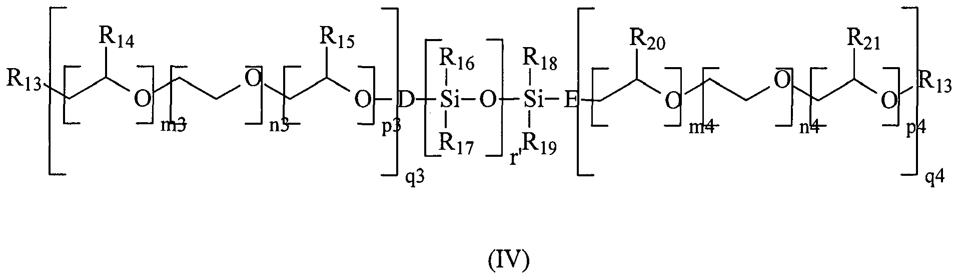 where:
- m₃, p₃, m₄ and p₄ represent a number of alkylene oxide units less than or equal to 150,
- n₃ and n₄ represent a number of ethylene oxide units less than or equal to 150,
- q₃ and q₄ represent an integer of at least 1 and such that 0 ≤ (m₃+n₃+p₃)q₃ ≤ 150 and 0 ≤ (m₄+n₄+p₄)q₄ ≤ 150,
- r' represents a number such that 1 ≤ r' ≤ 200,
- R₁₃ represents a radical containing an unsaturated polymerisable function, preferentially from the group of vinylics, or the group of acrylic, methacrylic, maleic, itaconic, crotonic, vinylphthalic esters, or the group of unsaturated urethanes such as acrylurethane, methacrylurethane, α-α' dimethyl-isopropenyl-benzylurethane, allylurethane, or the group of allylic or vinylic ethers, whether substituted or not or the group of ethylenically unsaturated amides or imides,
- R₁₄, R₁₅, R₂₀ and R₂₁ represent hydrogen or a methyl or ethyl radical,
- R₁₆, R₁₇, R₁₈ and R₁₉ represent linear or branched alkyl or aryl, or alkylaryl or arylalkyl groupings, having 1 to 20 carbon atoms, or their blends,
- D and E are groupings which may be present, which then represent a
hydrocarbon radical with 1 to 4 carbon atoms,
or among the blends of these molecules.

12. - The process according to one of claims 1 to 11, **characterised in that** said polymer has a molecular weight greater than 100,000 g/mole, , and preferentially greater than 120,000 g/mole.

13. - Thickened aqueous compositions, **characterised in that** they contain as thickener a polymer with at least one anionic monomer which is an organophosphate monomer, where said polymer has a molecular weight greater than 80,000 g/mole, preferentially greater than 100,000 g/mole, and very preferentially greater than 120,000 g/mole.

14. - The aqueous compositions according to claim 13, **characterised in that** the organophosphate monomer is chosen among molecules with the following formulas: 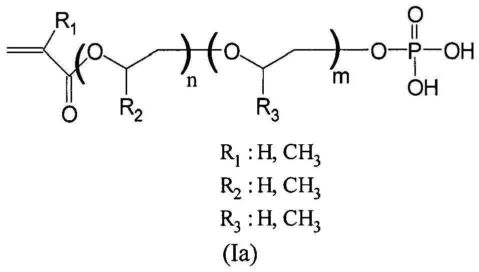 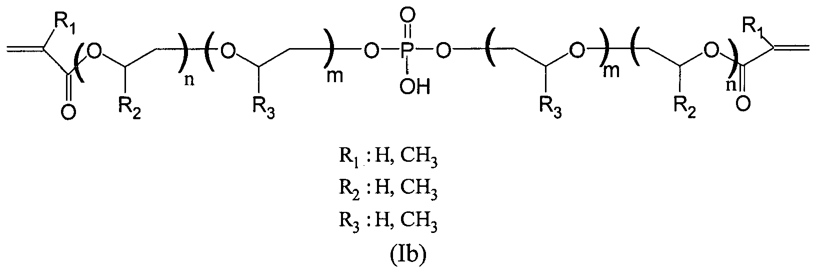 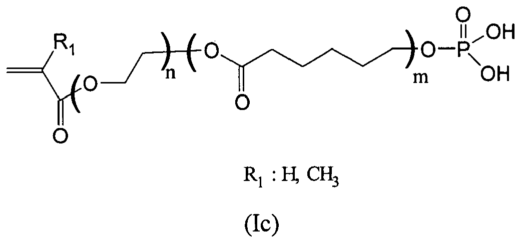 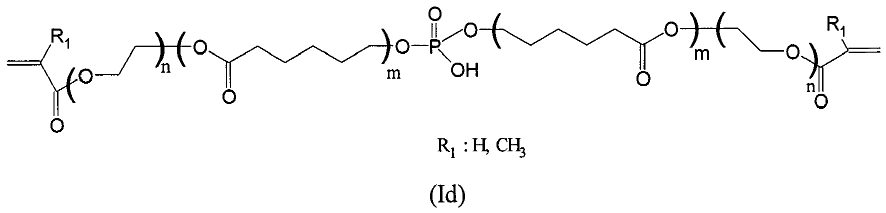 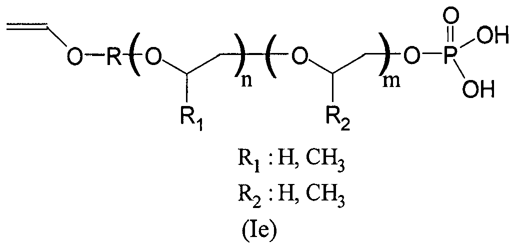 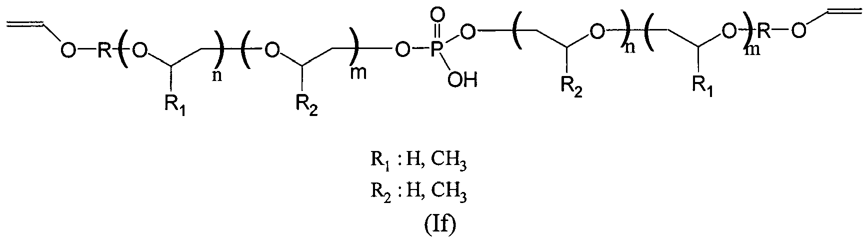 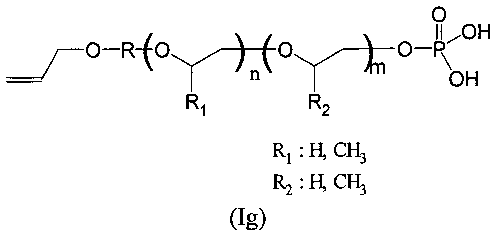 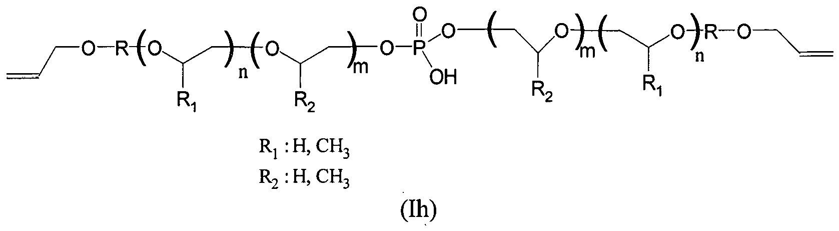 and their blends, with n designating an integer between 1 and 100, preferentially between 1 and 20, with m designating an integer between 0 and 100, preferentially between 0 and 20, and R designating an alkyl chain of 2 to 8 carbon atoms.

15. - The aqueous compositions according to one of claims 13 or 14, **characterised in that** they have a pH between 5 and 7, preferentially between 5 and 6.5, very preferentially between 5.5 and 6.

16. - The queous compositions according to one of claims 13 to 15, **characterised in that** said thickening polymer which they include, possibly contains:
a) at least one further anionic monomer different from the organophosphate monomer,
b) and/or at least one vinylic non-ionic monomer,
c) and/or at least one non-ionic monomer with a hydrophobic group,
d) and/or at least one organofluorate or organosililate monomer or their blends,
e) and/or at least one cross-linking monomer, i.e. a monomer having at least 2 polymerisable links, where said monomer is different from the organophosphate monomers of formulas (Ib), (Id), (If), and (Ih).

17. - The aqueous compositions according to claim 16, **characterised in that** said thickening polymer which theyinclude, contains, expressed inpercentage by weight of each of the constituents, from 0.01 to 100%, preferentially from 10 to 100%, very preferentially from 20 to 100% of the organophosphate monomer, and:
a) from 0 to 90% of at least one other anionic monomer differentfrom the organophosphate monomer,
b) from 0 to 50% of at least one non-ionic vinylic monomer,
c) from 0 to 20% of at least one non-ionic monomer with a hydrophobic group,
d) from 0 to 10% of at least one organofluorate or organosililate monomer or their blends,
e) from 0 to 5% of at least one cross-linking monomer, i.e. a monomer having at least 2 polymerisable links, where said monomer is different from the organophosphate monomers of formulas (Ib), (Id), (If), and (Ih).
where the sum of the percentages by weight of monomers constituting said polymer is equal to 100.

18. - The aqueous compositions, according to one of claims 16 or 17, **characterised in that** the anionic monomer a) of the thickening polymer which they contain is a monomer with ethylenic unsaturation and with a carboxylic group, chosen among the monomers with ethylenic unsaturation and with a monocarboxylic group, and is then preferentially acrylic, methacrylic, crotonic, isocrotonic or cinnamic acid, or their blends, or chosen among the hemiesters of diacids, and is then preferentially a monoester at C₁ to C₄ of the maleic or itaconic acids, or their blends, or chosen among the monomers with ethylenic unsaturation and with a dicarboxylic group in the acid or salified state, and preferentially among itaconic, maleic, fumaric or mesaconic acid, or their blends, or chosen among the anhydrides of carboxylic acids, and is then preferentially maleic anhydride.

19. - The aqueous compositions, according to one of claims 16 or 17, **characterised in that** the vinylic non-ionic monomer b) of the thickening polymer which they contain is chosen among esters, amides or nitriles of acrylic and methacrylic acid, and is then very preferentially chosen among the methyl, ethyl, butyl or 2-ethyl-hexyl acrylates or methacrylates, and their blends, or is chosen among acrylonitrile, vinyl acetate, styrene, methylstyrene, di-isobutylene, vinylpyrrolidone, vinylcaprolactame and their blends.

20. - The aqueous compositions, according to one of claims 16 or 17, **characterised in that** the non-ionic monomer with hydrophobic grouping c) of the thickening polymer which they contain is a monomer of formula (II): 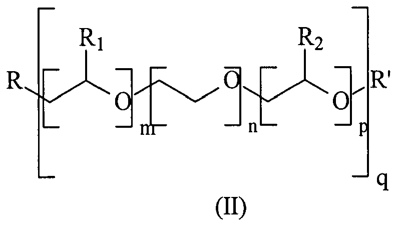 where:
- m and p represent a number of alkylene oxide units less than or equal to 150,
- n represents a number of ethylene oxide units less than or equal to 150,
- q represents an integer of at least 1 and such that 5 ≤ (m+n+p)q ≤ 150, and preferentially such that 15 ≤ (m+n+p)q ≤ 120,
- R₁ represents hydrogen or a methyl or ethyl radical,
- R₂ represents hydrogen or a methyl or ethyl radical,
- R represents a radical containing an unsaturated polymerisable function, preferentially from the group of vinylics, or the group of acrylic, methacrylic, maleic, itaconic, crotonic or vinylphthalic esters, or the group of unsaturated urethanes such as acrylurethane, methacrylurethane, α-α' dimethyl-isopropenyl-benzylurethane, allylurethane, or to the group of allylic or vinylic ethers, whether substituted or not, or the group of ethylenically unsaturated amides or imides,
- R' represents hydrogen or a hydrocarbonate radical having 5 to 50 atoms of carbon, and represents preferentially a hydrocarbonate radical having 12 to 50 carbon atoms, and very preferentially a hydrocarbonate radical with 16 to 36 carbon atoms,

21. - The aqueous compositions, according to one of claims 16 or 17, **characterised in that** the cross-linking monomer e) of the thickening polymer which they contain is chosen among the group constituted by ethylene glycol dimethacrylate, trimethylolpropanetriacrylate, allyl acrylate, the allyl maleates, methylene-bis-acrylamide, methylene-bis-methacrylamide, tetrallyloxyethane, the triallylcyanurates, the allyl ethers obtained from polyols such as pentaerythritol, sorbitol or sucrose, or chosen among the molecules of formula (IV): 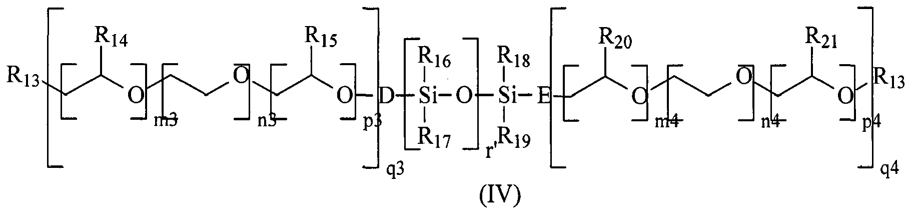 where:
- m₃, p₃, m₄ and p₄ represent a number of alkylene oxide units less than or equal to 150,
- n₃ and n₄ represent a number of ethylene oxide units less than or equal to 150,
- q₃ and q₄ represent an integer of at least 1 and such that 0 ≤ (m₃+n₃+p₃)q₃ ≤ 150 and 0 ≤ (m₄+n₄+p₄)q₄ ≤ 150,
- r' represents a number such that 1 ≤ r' ≤ 200,
- R₁₃ represents a radical containing an unsaturated polymerisable function, preferentially from the group of vinylics, or the group of acrylic, methacrylic, maleic, itaconic, crotonic, vinylphthalic esters, orthe group of unsaturated urethanes such as acrylurethane, methacrylurethane, α-α' dimethyl-isopropenyl-benzylurethane, allylurethane, or the group of allylic or vinylic ethers, whether substituted or not, or the group of ethylenically unsaturated amides or imides,
- R₁₄, R₁₅, R₂₀ and R₂₁ represent hydrogen or a methyl or ethyl radical,
- R₁₆, R₁₇, R₁₈ and R₁₉ represent linear or branched alkyl or aryl, or alkylaryl or arylalkyl groupings, having 1 to 20 carbon atoms, or their blends,
- D and E are groupings which may be present, which then represent a
hydrocarbon radical having 1 to 4 carbon atoms,
or among the blends of these molecules.

22. - The aqueous compositions according to one of claims 16 to 21, **characterised in that** they are cosmetic compositions, pharmaceutical, compositions with a hydraulic binder base, and are then preferentially concretes, cements, mortars, cement slips or slags, detergent compositions, paper coatings colors, paints.
